(19) Europäisches Patentamt
European Patent Office
Office européen des brevets

(11) Publication number: **0 386 892 A2**

(12) # EUROPEAN PATENT APPLICATION

(21) Application number: 90301538.6

(22) Date of filing: 14.02.90

(51) Int. Cl.5: C07D 231/54, C07D 491/04, A01N 43/56, A01N 43/90, //(C07D491/04,311:00,231:00)

(30) Priority: 09.03.89 US 321075
14.09.89 US 407065

(43) Date of publication of application:
12.09.90 Bulletin 90/37

(84) Designated Contracting States:
GR

(71) Applicant: E.I. DU PONT DE NEMOURS AND COMPANY
1007 Market Street
Wilmington Delaware 19898(US)

(72) Inventor: Harrison, Charles Richard
137 Aspen Drive
Newark, Delaware 19702(US)
Inventor: Lahm, George Philip
148 Fairhill Drive
Wilmington, Delaware 19808(US)
Inventor: Shapiro, Rafael
1415 Fresno Road
Wilmington, Delaware 19803(US)

(74) Representative: Hildyard, Edward Martin et al
Frank B. Dehn & Co. European Patent Attorneys Imperial House 15-19 Kingsway
London WC2B 6UZ(GB)

(54) **Arthropodicidal tetrahydrobenzopyranopyrazoles.**

(57) This invention concerns substituted tetrahydrobenzopyranopyrazoles of the formula

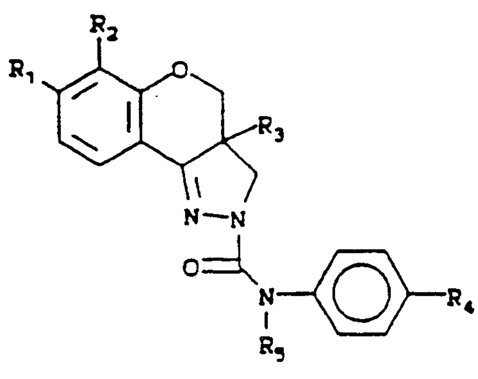

wherein $R_1$ to $R_5$ are substituents, or $R_1$, $R_2$ and $R_5$ may independently be hydrogen; intermediates thereto, agricultural compositions containing the tetrahydrobenzopyranopyrazoles and a method for using them to control arthropods in agronomic and nonagronomic environments. Also disclosed is a process for preparing the tetrahydrobenzopyranopyrazoles by cyclization of phosphorus-containing starting reactants in the presence of a halogenating agent and base.

EP 0 386 892 A2

# ARTHROPODICIDAL TETRAHYDROBENZOPYRANOPYRAZOLES

## CROSS-REFERENCE TO RELATED APPLICATIONS

This is a continuation-in-part of U.S. Serial No. 07/407,065 filed on September 14, 1989 which is a continuation-in-part of U.S. Serial No. 07/321,075 filed on March 9, 1989.

## BACKGROUND OF THE INVENTION

This invention pertains to substituted tetrahydrobenzopyranopyrazoles, agricultural compositions containing them and a method of use to control arthropods in agronomic and nonagronomic environments. This invention also pertains to a process for preparing compounds of this invention.

WO 88/07994 discloses insecticidal substituted indazoles that broadly encompass those of the instant invention. However, there is no specific disclosure of any of the compounds of this invention.

Bull. Chem., Soc. Jap. 55, pages 2450 to 2455 (1982) discloses a process for 1,3-dipolar cycloadditions of arylhydrazones.

## SUMMARY OF THE INVENTION

This invention pertains to tetrahydrobenzopyranopyrazoles of Formula I, including all geometric and stereoisomers, agronomically and nonagronomically suitable salts thereof, agronomically and nonagronomically useful compositions containing them and their use for the control of arthropods in both agronomic and nonagronomic environments. The term "compounds" will be understood to include all such isomers and salts thereof. The compounds are:

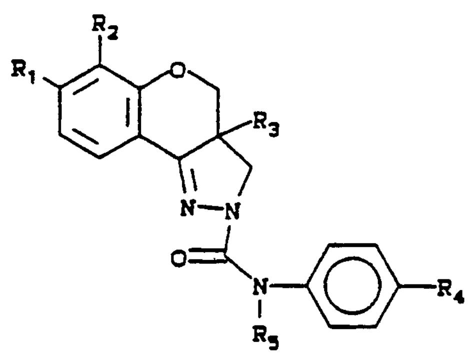

(I)

wherein

$R_1$ is selected from H, F, Cl and $CF_3$;

$R_2$ is selected from H and F, one of $R_1$ or $R_2$ being H but not both;

$R_3$ is selected from $C_1$ to $C_6$ alkyl, $C_3$ to $C_6$ cycloalkyl, $C_4$ to $C_7$ cycloalkylalkyl, $CO_2CH_3$, phenyl optionally substituted with F or Cl in the para-position and benzyl optionally substituted with F or Cl in the para-position;

$R_4$ is selected from Cl, Br, $CF_3$ and $OCF_3$; and

$R_5$ is selected from H, C(O)$CH_3$ and C(O)O$CH_3$;

provided that:

(i) when $R_1$ is F, $R_4$ is Br and $R_5$ is H, then $R_3$ is other than $CO_2CH_3$; and

(ii) when $R_1$ is Cl, $R_4$ is Cl and $R_5$ is H, then $R_3$ is other than n-propyl.

This invention also concerns intermediates of Formula IV (salts or free base) wherein $R_1$ to $R_3$ are as defined above and Z as defined below. This invention also pertains to the novel process of Scheme 1 for

preparing compounds of Formula I by intramolecular cyclization of olefin II to tetrahydropyrazole III in step (i), removal of the phosphorus moiety in step (ii), and addition of the C(O)NH-aryl moiety in step (iii):

SCHEMA 1

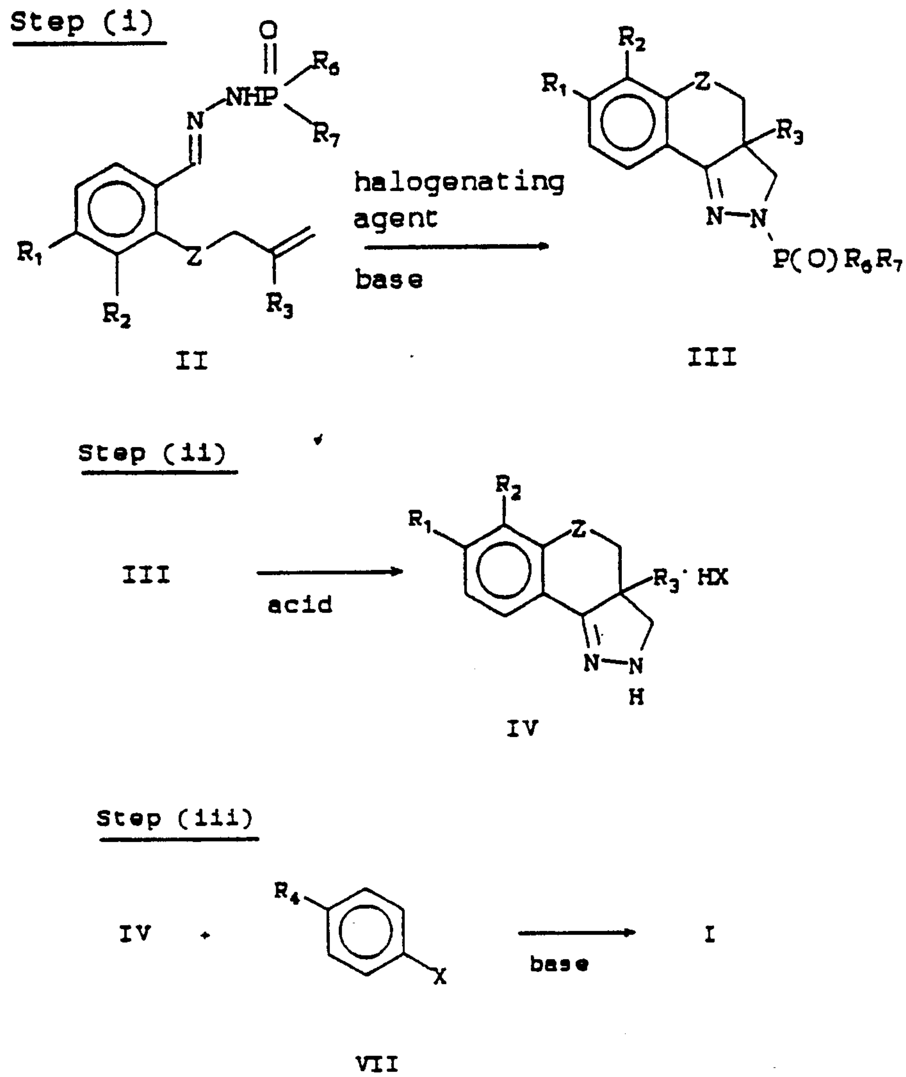


wherein

$R_1$, $R_2$, $R_3$, $R_4$ and $R_5$ are as described earlier;

$R_6$ and $R_7$ are independently selected from $C_1$ to $C_4$ alkyl, $C_1$ to $C_4$ alkoxy and phenyl optionally substituted with $C_1$ to $C_3$ alkyl or halogen; or, $R_6$ and $R_7$ can be taken together to form a 5- or 6-membered ring optionally substituted with $C_1$ to $C_2$ alkyl;

Z is $CH_2$ or O; and

X is NCO or NHCOF.

Preferred Compounds A are the compounds of Formula I wherein $R_3$ is selected from $C_1$-$C_6$ alkyl, $CO_2CH_3$, phenyl optionally substituted with F or Cl in the para-position, and benzyl optionally substituted with F or Cl in the para-position.

Preferred Compounds B are Compounds A wherein $R_3$ is selected from $C_1$-$C_6$ alkyl, $CO_2CH_3$, and

phenyl optionally substituted with F or Cl in the para-position.

Preferred Compounds C are Compounds A of Formula I wherein $R_3$ is selected from $C_3$-$C_6$ cycloalkyl and $C_4$-$C_7$ cycloalkylalkyl.

Preferred Compounds D are Compounds A wherein $R_3$ is selected from $CH_3$, isopropyl, and $CO_2CH_3$; and $R_4$ is selected from $CF_3$, $OCF_3$, and $R_5$ is H.

Specifically preferred are the following compounds of preferred Compounds D:

E methyl 7-chloro-2,3,3a,4-tetrahydro-2-[[[4-(trifluoromethyl)phenyl]amino]carbonyl][1]benzopyrano-[4,3-c]-pyrazole-3a-carboxylate,

F 7-chloro-2,3,3a,4-tetrahydro-3a-methyl-N-[4-(trifluoromethyl)phenyl][1]benzopyrano-[4,3-c]pyrazole-2-carboxamide,

G 7-chloro-2,3,3a,4-tetrahydro-3a-(1-methylethyl)-N-[4-(trifluoromethyl)phenyl][1]benzopyrano[4,3-c]-pyrazole-2-carboxamide,

H 6-fluoro-3a-(4-fluorophenyl)-2,3,3a,4-tetrahydro-N-[4-(trifluoromethyl)phenyl][1]benzopyrano[4,3-c]-pyrazole-2-carboxamide,

I methyl 2,3,3a,4-tetrahydro-7-(trifluoromethyl)-2-[[[4-(trifluoromethyl)phenyl]amino]carbonyl][1]benzopyrano-[4,3-c]pyrazole-3a-carboxylate, and

J methyl 2,3,3a,4-tetrahydro-7-(trifluoromethyl)-2-[[[4-(trifluoromethoxy)phenyl]amino]carbonyl][1]-benzopyrano[4,3-c]pyrazole-3a-carboxylate.

## DETAILS OF THE INVENTION

Alternative methods for preparing compounds of Formula I are summarized in Schemae 2 and 3.

### SCHEMA 2

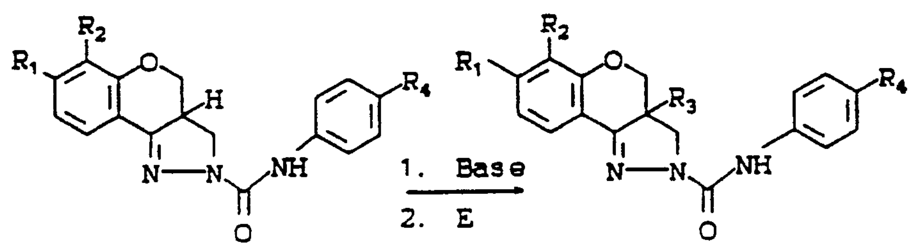


Ia　　　　Ib

wherein:
$R_3$ is $C_1$-$C_4$ alkyl or $CO_2CH_3$;
E is an electrophilic reagent such as a $C_1$-$C_4$ alkyl iodide or $ClCO_2CH_3$; and
$R_1$, $R_2$ and $R_4$ are as already defined.

SCHEMA 3

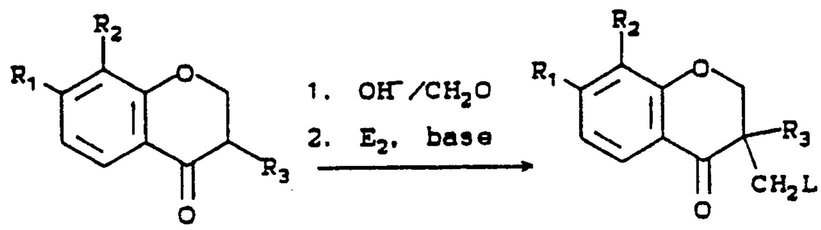


V VI

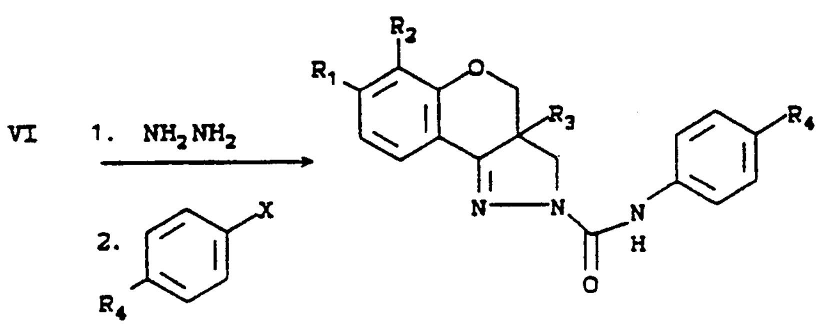


VII Ic

wherein:

$E_2$ is an electrophilic reagent, for activating alcohols toward nucleophilic substitutions such as $SOCl_2$, $PBr_3$, $CH_3SO_2Cl$ and the like;

L is a leaving group such as chloride, bromide, tosylate or mesylate;

X is NCO or NHCOF;

$R_3$ is phenyl optionally substituted in the 4-position by fluorine or chlorine; and

$R_1$, $R_2$, and $R_4$ are as already defined.

Starting reactant II for the process of this invention can be made by reacting aldehyde VIII with phosphoryl hydrazide IX.

SCHEMA 4

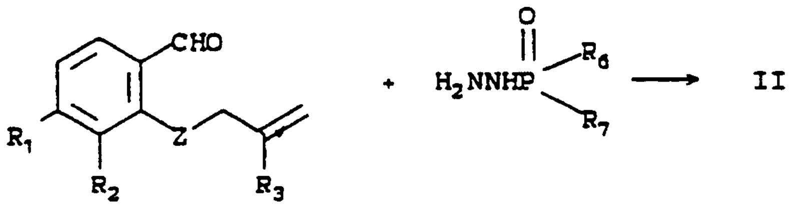


VIII IX

This reaction is typically carried out in an inert solvent such as ethanol or dichloromethane containing 0 to about 5 mol percent of an acid catalyst such as acetic or p-toluenesulfonic. Approximately equimolar amounts of the two components, VIII and IX, are mixed in the solvent and catalyst, if any, and maintained at a temperature between about 0 to 30 °C for a period of about 5 to 150 minutes. Compound II, that will be used in the process of this invention, can crystallize from the reaction solvent; if not, it can be isolated by evaporating the bulk of the solvent, diluting with water, filtering the product, and drying under vacuum. In the event that the product does not crystallize from the aqueous medium, it can be extracted into an inert solvent such as dichloromethane. After drying the organic extract with a suitable drying agent such as sodium sulfate, it can be used in step (i) of this invention, or concentrated and optionally purified by recrystallization or chromatography on silica gel. Suitable solvent mixtures for recrystallization include aqueous ethanol or methanol, ether-hexanes, or ethyl acetate-hexanes. The latter mixture can be used for silica gel chromatography.

Step (i)

This reaction can be conducted by gradually adding a base, such as triethylamine, to a solution or suspension of II and a moderately reactive halogenating agent, such as N-chlorosuccinimide, in an inert, dry solvent such as ether, dichloromethane, toluene, and the like.

Alternatively, the halogenating agent can be gradually added to a mixture of the base and the compound of Formula II. In either case, optimum yields of III are obtained when the temperature (between -20 and 40 °C) and concentration (between about 0.0001 and 3M) are as low as possible and when the component being added to the reaction mixture is added gradually. Thus, conditions should be chosen so that the intramolecular cycloaddition of the postulated intermediate, X, is favored over bimolecular side reactions, such as oligomerization. Intermediate, X, is believed to be:

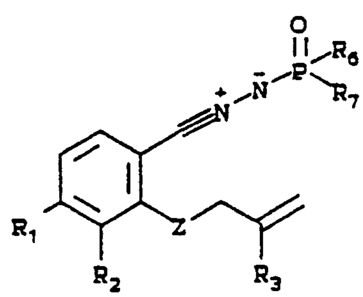

X

The reagent, N-chlorosuccinimide, is preferred but other moderately reactive halogenating agents such as N-bromosuccinimide, t-butyl hypochlorite, iodine, or sulfuryl chloride can also be used. The base of choice generally depends upon the reactivity of the halogen source. For example, whereas a trialkylamine is suitable for use with N-haloamides, an alkaline-earth or alkali-metal carbonate, bicarbonate, or oxide may be preferable when using the more reactive t-butyl hypochlorite. For optimum yields, it is important to maintain anhydrous conditions.

Isolation and purification of III is generally accomplished by washing an ether solution thereof to remove water-soluble by-products, e.g., succinimide and trialkylamine hydrohalides, drying the purified extract with an inert inorganic salt such as magnesium sulfate or another suitable drying agent, concentration of the filtered ether solution, and crystallization from a suitable organic solvent or solvent mixture, e.g., ether and hexane. If III fails to crystallize it can nevertheless be carried on to step (ii) or, if not sufficiently pure, it can be chromatographed on a silica gel column using a suitable mixture of solvents such as ethyl acetate and hexanes.

Step (ii)

This step is performed by reacting a compound of Formula III with about 0.9 to 5.0 equivalents of a strong acid such as HCl, $H_2SO_4$, or p-toluenesulfonic in a suitable solvent such as tetrahydrofuran or a lower alkanol optionally containing 0.1 to 10 equivalents of water at a temperature of between about 20 to 100°C. After an appropriate contact time, usually between 1 to 48 hours, the mixture is cooled to about 0-5°C and the precipitated intermediate IV is collected by filtration. In cases where IV fails to crystallize, it can be precipitated by the addition of ether (optionally after concentration to remove excess reaction solvent). Alternatively, in cases where IV fails to crystallize, the reaction medium can be neutralized with an aqueous solution of an inorganic base such as sodium bicarbonate. The reaction solvent is then removed by evaporation and the free base of IV is extracted into an organic solvent such as ether, ethyl acetate or dichloromethane. The organic extracts are then used directly in step (iii).

Step (iii)

This step is carried out by reacting the salt of IV with an aromatic isocyanate in a slurry of an inorganic base such as potassium carbonate, about 0.5 to 1.1 equivalents, in a non-polar, organic solvent such as ether or dichloromethane containing about 1 to 50 equivalents of water. The isocyanate is employed at about 1.0 to 1.1 equivalents. The reaction is best carried out at a temperature between about 0 and 30°C and is usually complete within one hour after addition of the isocyanate. The product is typically isolated by drying the organic phase with a suitable inorganic salt, filtering and evaporating the solvent. In cases where IV has been isolated as its free base it can be treated directly in the extraction solvent with an approximate equimolar amount of the aromatic isocyanate, followed by evaporation of the solvent. Washing the residue with a non-polar solvent such as hexanes and/or ether provides the product of Formula I in pure form as a crystalline solid.

Alternatively, IV can be treated with two equivalents of a base, typically triethylamine, and one equivalent of a carbamoyl fluoride of Formula VII in a solvent such as dichloromethane or acetonitrile. Dilution with water and evaporation of solvent (for water-immiscible solvents), or filtration (for water-miscible solvents) affords the product of Formula I which can be purified by washing with a solvent such as diethyl ether.

The synthesis of starting materials of Formulas VIII and IX can be carried out by analogy with procedures documented in the art.

Alternatively, compounds of Formula IV can be obtained by decomposition of sulfonylhydrazides containing an unsaturated side chain (Formula XI) and isomerization of the cycloaddition product (Formula XII). The thermal, base catalyzed decomposition of sulfonylhydrazides containing unsaturation has been studied by Kimura (Chem. Ber., 1967, 100, 2710) and Padwa (J. Org. Chem., 1980, 45, 3756). Conditions for the decomposition of sulfonylhydrazides in general (Bamford-Stevens Reaction) have been reviewed by Shapiro (Org. Reactions, 23, 453). The conversion of compounds of Formula XI to compounds of Formula IV can be accomplished with a variety of bases. Such bases include sodium hydride, alkyl lithiums, sodium alcoholates, and potassium alcoholates. Among these, sodium hydride is preferred when aprotic solvents are utilized. Other applicable solvents include ethylene glycol, lower alcohols, aromatic hydrocarbons such as benzene, toluene, and xylenes, and various aprotic solvents such as diglyme. Suitable solvents are the

aromatic hydrocarbons, with toluene being the most preferred. In general, the reaction must be heated in the range from 50-110°C in order to obtain the desired cycloaddition. However, in some cases, lower or higher temperatures may be applicable. Alternatively, the compounds of Formula XII can be obtained by treatment of the hydrazones of Formula XI with Lewis acids such as boron trifluoride in aprotic or hydrocarbon solvents and separate isomerization with a base such as sodium hydroxide or potassium carbonate.

SCHEMA 5

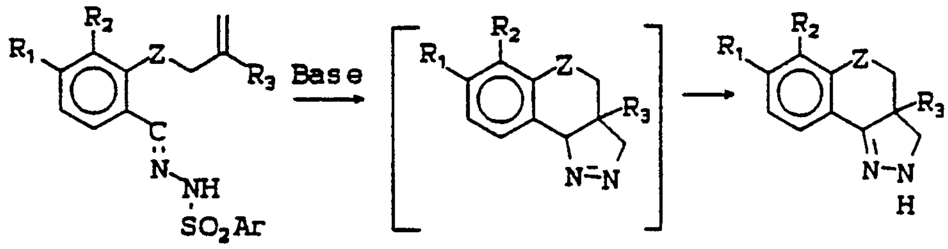


XI XII IV

wherein:
$R_1$, $R_2$, Z, and $R_3$ are as previously defined.

Examples 1 to 6 illustrate the invention in more detail.

EXAMPLE 1

Step A

4-Chloro-2-hydroxybenzaldehyde

Modification of a procedure described by G. Casiraghi et al., (J. Chem. Soc., Perkin Transac. I., 1978, 318), was utilized in the preparation of substituted salicylaldehydes. To a mixture of 26 g of 3-chlorophenol dissolved in 200 mL of toluene at 10°C was added dropwise 100 mL of a 2M solution of ethylmagnesium chloride in diethyl ether. The ether was distilled off and the reaction mixture was cooled to room temperature and 26.0 g of 1,3-dimethyl-3,4,5,6-tetrahydro-2(1H)-pyrimidinone (DMPU) was added followed by 15.0 g of paraformaldehyde. The reaction mixture was heated at 70°C for 18 h, cooled to 10°C and a solution of 20 mL of concentrated HCl in 100 g of ice was added followed by 200 mL of hexane. The organic phase was separated and washed twice with 100 mL of water, once with 100 mL of brine, dried over anhydrous $MgSO_4$, filtered and concentrated under vacuum to afford a viscous oil. This was taken up in 150 mL of ethanol and a solution of 15.0 g of copper (II) acetate dissolved in 150 mL of $H_2O$ was added dropwise. The resulting precipitate was filtered, washed with water and then with ether and added to a solution of 200 mL of ether and 20 mL of concentrated $H_2SO_4$ in 100 g of ice. The mixture was stirred until all solids were dissolved, 100 mL of hexane was added, and the organics were washed with 100 mL of $H_2O$, then 100 mL of brine, dried over anhydrous $MgSO_4$, filtered and concentrated under vacuum to afford 15.0

g of a yellow solid, m.p. 47° C to 48° C.
$^1H$ NMR ($CDCl_3$) δ 6.98 (d, 1H), 7.01 (s, 1H), 7.50 (d, 1H), 9.85 (s, 1H), 11.18 (s, 1H). IR (Nujol) 3300-2800, 1670 $cm^{-1}$.

Step B

Methyl 2-[(5-Chloro-2-formylphenoxy)methyl]-2-propenoate

To 75 ml of acetone was added 4.4 g of $K_2CO_3$, 5.0 g of 4-chloro-2-hydroxybenzaldehyde and 5.7 g of methyl (2-bromomethyl)-2-propenoate. The mixture was heated at reflux for 30 minutes, cooled, filtered and the solution was concentrated to afford a white solid. The solid was dissolved in 100 mL of ether and washed with 100 mL of $H_2O$ and 100 mL of brine, dried over anhydrous $MgSO_4$, filtered and concentrated under vacuum to afford 7.1 g of a white powder, m.p. 100° C to 102° C.
$^1H$ NMR ($CDCl_3$) δ 3.84 (s, 3H), 4.86 (s, 2H), 6.04 (s, 1H), 6.49 (s, 1H), 7.03 (s, 1H), 7.05 (d, 1H), 7.81 (d, 1H), 10.43 (s, 1H). IR (Nujol) 1735, 1685 $cm^{-1}$.

Step C

Methyl 2[[5-chloro-2-[[(diethylphosphinyl)hydrazono]methyl]phenoxy]methyl]-2-propenoate

A mixture of 6.0 g of the product isolated from Step B, 4.75 g of diethyl phosphorohydrazidate (A. Zwierzak, Synthesis, 1976, 835) and 100 mg of p-toluenesulfonic acid in 50 mL of methanol was heated at reflux for 30 minutes. The reaction mixture was cooled, poured into 200 ml of $H_2O$, extracted with ethyl acetate, washed with 100 mL of $H_2O$ and then with 100 mL of brine, dried over anhydrous $MgSO_4$, filtered and concentrated under vacuum to afford 8.3 g of a white powder, m.p. 134° C to 136° C.
$^1H$ NMR ($CDCl_3$) δ 1.40 (t, 6H), 3.83 (s, 3H), 4.20 (m, 4H), 4.77 (s, 2H), 5.99 (s, 1H), 6.48 (s, 1H), 6.9 (d, 1H), 6.97 (d, 1H), 7.8 (d, 1H), 8.06 (s, 1H). IR (Nujol) 3140, 1730 $cm^{-1}$.

Step D

Methyl 7-chloro-2-(diethylphosphinyl)-2,3,3a,4-tetrahydro[1]benzopyrano[4,3-c]pyrazole-3a-carboxylate

To a solution of 5.0 g of the product obtained in Step C and 1.8 g of N-chlorosuccinimide in 100 mL of $CH_2Cl_2$ at room temperature was added a solution of 1.6 g of triethylamine in 25 mL of $CH_2Cl_2$, dropwise, over 2 hours. The reaction mixture was washed with 100 mL of 5% HCl and 100 mL of $H_2O$, dried over anhydrous $MgSO_4$, filtered and concentrated under vacuum to afford 4.04 g of a viscous oil.
$^1H$ NMR ($CDCl_3$) δ 1.35 (t, 6H), 3.43 (dd, 1H), 3.72 (s, 3H), 4.0-4.3 (m, 6H), 4.91 (d, 1H), 6.9-7.0 (m, 2H), 7.76 (d, 1H).

Step E

Methyl 7-chloro-2,3,3a,4-tetrahydro-2-[[[4-(trifluoromethyl)phenyl]amino]carbonyl][1]benzopyrano[4,3-c]pyrazole-3-a-carboxylate

A mixture of 4.0 g of the product obtained in Step D and 2.0 g of p-toluenesulfonic acid in 50 mL of $CH_3OH$ was heated at reflux for 18 hours. The reaction mixture was cooled, concentrated under vacuum

and dissolved in 100 mL of $H_2O$ and 3.0 g of $K_2CO_3$, 50 mL of EtOAc and 2.06 g of α,α,α-trifluoro-p-tolylisocyanate was added. The reaction mixture was stirred for 30 minutes at room temperature. The aqueous phase was extracted with ethyl acetate, washed with 100 mL of $H_2O$, 100 mL of brine, dried over anhydrous $MgSO_4$, filtered and concentrated under vacuum to afford a yellow solid. Trituration with ether and filtration afforded 3.6 g of an off-white powder, m.p. = 214 °C to 215 °C.
$^1$H NMR ($CDCl_3$) δ 3.76 (s, 3H), 3.80 (d, 1H), 4.20 (d, 1H), 4.50 (d, 1H), 5.03 (d, 1H), 7.05 (m, 2H), 7.6 (ABq, 4H), 7.79 (d, 1H), 8.12 (bs, 1H). IR (Nujol) 3380, 1725, 1700 cm$^{-1}$.

## EXAMPLE 2

### Step A

#### 4-Chloro-2-[(2-methyl-2-propenyl)oxy]benzaldehyde

To a solution of 3.60 g of potassium tert-butoxide dissolved in 75 mL of THF and cooled to 0 °C was added dropwise a solution of 5.0 g of the product obtained in Step A of Example 1 in 25 mL of THF. The reaction mixture was allowed to warm to room temperature, stirred for 15 minutes and 3.20 g of 3-chloro-2-methylpropene was added. The reaction mixture was heated at reflux for 18 hours, cooled, poured into 200 mL of 5% HCl, extracted with ether, washed with 100 mL of $H_2O$, 100 mL of 1 N NaOH, and 100 mL of brine, dried over anhydrous $MgSO_4$, filtered, and concentrated under vacuum to afford 6.2 g of a white powder.
$^1$H NMR ($CDCl_3$) δ 1.86 (s, 3H), 4.53 (s, 2H), 5.06 (s, 1H), 5.12 (s, 1H), 7.00 (m, 2H), 7.79 (d, 1H), 10.5 (s, 1H). IR (Nujol) 1700 cm$^{-1}$.

### Step B

#### Diethyl[[4-chloro-2-[(2-methyl-2-propenyl)oxy]phenyl]methylene]phosphorohydrazidate

Application of the procedure described in Step C of Example 1 afforded 3.4 g of a white powder, m.p. = 100 °C to 101 °C.
$^1$H NMR ($CDCl_3$) δ 1.30 (m, 6H), 1.84 (s, 3H), 4.20 (m, 4H), 4.45 (s, 2H), 5.02 (s, 1H), 5.08 (s, 1H), 6.80 (d, 1H), 6.84 (s, 1H), 6.95 (d, 1H), 7.8 (d, 1H), 8.1 (s, 1H). IR (Nujol) 3120, 1600 cm$^{-1}$.

### Step C

#### Diethyl(7-chloro-2,3,3a,4-tetrahydro-3a-methyl-[1]benzopyrano[4,3-c]pyrazol-2-yl)phosphonate

Application of the procedure described in Step D of Example 1 afforded 3.1 g of a viscous oil. Trituration with hexanes afforded a solid, m.p. = 63 °C to 66 °C.
$^1$H NMR ($CDCl_3$) δ 1.35 (m, 9H), 3.45 (dd, 1H), 3.75 (d, 1H), 4.20 (m, 5H), 4.38 (d, 1H), 6.90 (m, 2H), 7.72 (d, 1H).

### Step D

#### 7-Chloro-2,3,3a,4-tetrahydro-3a-methyl-N-[4-(trifluoromethyl)phenyl][1]benzopyrano[4,3-c]pyrazole-2-carboxamide

Application of the procedure described in Step E of Example 1 afforded 0.9 g of an off-white powder, m.p. = 182° C to 183° C.
$^1$H NMR ($CDCl_3$) δ 1.41 (S, 3H), 3.63 (d, 1H), 4.03 (d, 1H), 4.18 (d, 1H), 4.42 (d, 1H), 7.0 (m, 2H), 7.5-7.8 (m, 6H), 8.12 (s, 1H). IR (Nujol) 3400, 1690 $cm^{-1}$.

EXAMPLE 3

Step A

4-Chloro-2-(3-methyl-2-methylenebutoxy)-benzaldehyde

To a solution of 8.8 g (0.064 mol) of potassium carbonate in 100 mL of dimethylformamide was added 10.0 g (0.064 mol) of 4-chlorosalicylaldehyde and 12.5 g (0.077 mol) of 2-(bromomethyl)-3-methyl-1-butene. The reaction mixture was heated at 70° C for 2 h, cooled to room temperature and poured into 600 mL ice/water. The crude mixture was extracted with ethyl acetate (5 x 100 mL). The ethyl acetate was washed with water (100 mL) and brine (100 mL), dried over anhydrous magnesium sulfate, filtered and concentrated under reduced pressure to afford 14.0 g of a yellow oil.
200 MHz, $^1$H NMR ($CDCl_3$) δ 1.15 (d, 6H), 2.4 (m, 1H), 4.61 (s, 2H), 5.08 (s, 1H), 5.15 (s, 1H), 6.99 (s, 1H), 7.02 (d, 1H), 7.80 (d, 1H), 10.5 (s, 1H). IR (neat) 1685 $cm^{-1}$.

Step B

Diethyl [[4-chloro-2-(3-methyl-2-methylenebutoxy)phenyl]methylene)phosphorohydrazidate

To a solution of 11.8 g (0.070 mol) of diethyl phosphorohydrazidate in 140 mL ether was added 14.0 g (0.058 mol) of the product obtained in Step A. The reaction was stirred at room temperature for 30 minutes. The resultant precipitate was filtered and washed with ether (50 mL) and dried to afford 15.0 g of a white solid, m.p. 103° C to 106° C.
200 MHz, $^1$H NMR ($CDCl_3$) δ 1.15 (d, 6H), 1.35 (t, 6H), 2.45 (m, 1H), 4.2 (m, 4H), 4.52 (s, 2H), 5.06 (s, 1H), 5.10 (s, 1H), 6.9 (m, 3H), 7.80 (d, 1H), 8.1 (s, 1H).
IR (nujol) 3140, 1600, 1020 $cm^{-1}$.

Step (C)

7-Chloro-2,3,3a,4-tetrahydro-3a-methyl-N-[4-(trifluoromethyl)phenyl][1]benzopyrano[4,3-c]pyrazole-2-carboxamide

To a solution of 6.8 g (0.017 mol) of the product obtained in Step B dissolved in 90 mL methylene chloride was added 2.8 g (0.021 mol) of N-chlorosuccinimide. The reaction mixture was stirred at room temperature and 2.7 g (0.026 mol) of triethylamine dissolved in 20 mL methylene chloride was added dropwise over 1 hour. The reaction mixture was extracted with a 5% HCl solution (100 mL) and washed with water (100 mL), dried over anhydrous magnesium sulfate, filtered and concentrated under reduced pressure to afford 6.3 g of a red oil. This oil was taken up in 60 mL of methanol and 4.9 mL of concentrated HCl was added. The reaction mixture was heated at reflux for 3 h, cooled and concentrated under reduced pressure. 200 mL of saturated sodium bicarbonate solution was added and the reaction mixture was extracted with ethyl acetate (3 x 100 mL). The ethyl acetate was washed with brine (100 mL) dried over anhydrous

magnesium sulfate and filtered into a flask containing 3.0 g (0.016 mol) of α,α,α-trifluoro-p-tolylisocyanate. The reaction mixture was stirred at room temperature for 30 minutes and concentrated under reduced pressure to afford a yellow solid. The yellow solid was chromatographed on silica gel using tetrahydrofuran (20%) and hexanes (80%) as the eluent which afforded 6.4 g of a white solid, m.p. 150 °C to 152 °C.
200 MHz, $^1$H NMR ($CDCl_3$) δ 0.82 (d, 3H), 1.1 (d, 3H), 2.11 (m, 1H), 3.45 (d, 1H), 4.10 (d, 1H), 4.15 (d, 1H), 4.63 (d, 1H), 6.98 (s, 1H), 7.0 (d, 1H), 7.60 (ABq, 4H), 7.75 (d, 1H), 8.11 (s, 1H).
IR (nujol) 3390, 1685 $cm^{-1}$.

## EXAMPLE 4

### Step A

#### 1-[1-(Bromomethyl)ethenyl-4-fluorobenzene

To a mixture of 10.0 g (0.073 mol) of 4-fluoro-α-methylstyrene and 15.4 g (0.080 mol) of p-toluenesulfonic acid monohydrate dissolved in 200 mL tetrahydrofuran at room temperature was added 13.7 g (0.077 mol) of N-bromosuccinimide all at once. The reaction mixture was heated at reflux for 5 minutes, cooled to room temperature and 100 mL hexanes was added. The organic phase was extracted with 200 mL of saturated $NaHCO_3$ (aq.) and brine (200 mL), dried over anhydrous magnesium sulfate, filtered and concentrated under reduced pressure to afford an oil. The oil was filtered through silica gel using hexane as eluent. Concentration of the filtrate under reduced pressure afforded 9.1 g of a colorless oil.
200 MHz, $^1$H NMR ($CDCl_3$) δ 4.35 (s, 2H), 5.47 (s, 1H), 5.50 (s, 1H), 7.05 (m, 2H), 7.45 (m, 2H).

### Step B

#### 3-Fluoro-2-[[-(4-fluorophenyl)-2-propenyl]oxy]benzaldehyde

Application of the procedure described in Step B of Example 1 to the product obtained in Step A above afforded isolation of 9.1 g of a white solid, m.p. 52 °C to 53 °C.
200 MHz, $^1$H NMR ($CDCl_3$) δ 5.16 (s, 2H), 5.39 (s, 1H), 5.52 (s, 1H), 7.0-7.45 (m, 6H), 7.55 (d, 1H), 9.99 (s, 1H).
IR (nujol) 1685 $cm^{-1}$.

### Step C

#### Diethyl [[3-fluoro-2-[[2-(4-fluorophenyl)-2-propenyl]oxy]phenyl]methylene]phosphorohydrazidate

Application of the procedure described in Step C of Example 1 afforded isolation of 9.98 g of a white solid, m.p. 102 °C to 104 °C.
200 MHz, $^1$H NMR ($CDCl_3$) δ 1.34 (t, 6H), 4.15 (m, 4H), 5.02 (s, 2H), 5.37 (s, 1H), 5.50 (s, 1H), 6.45 (d, 1H), 7.05 (m, 4H), 7.50 (m, 4H).
IR (nujol) 3120, 1160, 1020 $cm^{-1}$.

### Step D

6-Fluoro-3a-(4-fluorophenyl)-2,3,3a,4-tetrahydro-N-[4-(trifluoromethyl)phenyl][1]benzopyrano[4,3-c]pyrazole-2-carboxamide

Application of the procedures described in Step D and E of Example 1 to the product obtained from Step C afforded 7.0 g of a white solid, m.p. 185° C to 187° C.
200 MHz, $^1$H NMR ($CDCl_3$) δ 3.96 (d, 1H), 4.26 (d, 1H), 4.48 (d, 1H), 5.20 (d, 1H), 7.0-7.15 (m, 4H), 7.3 (m, 2H), 7.7 (m, 5H), 8.22 (bs, 1H).
IR (nujol) 3400, 1679 $cm^{-1}$.

EXAMPLE 5

Step A

2-Nitro-4-(trifluoromethyl)benzonitrile

A mixture of 45.0 g (0.20 mol) of 1-chloro-2-nitro-4-(trifluoromethyl)benzene 39.4 g (0.44 mol) cuprous cyanide 6.4 g (0.040 mol) bromine, 3.2 g (0.040 mol) of pyridine, and 200 mL of dimethylformamide was heated at reflux for 4 hours. After cooling the mixture to 20° C, 200 mL of water, 75 g of ferric chloride and 60 mL of concentrated hydrochloric acid were added. The mixture was extracted with three 500 mL portions of toluene. The combined toluene extracts were washed with water, dried over anhydrous magnesium sulfate, and the toluene was evaporated. The residue (33 g) crystallized on standing.

Recrystallization from a mixture of chlorobutane and cyclohexane yielded a colorless solid melting at 43-45° C. The IR (neat) spectra displays a strong bond at 4.5 μ.

Step B

2-Methoxy-4-(trifluoromethyl)benzonitrile

Twenty-five percent sodium methoxide in methanol (24.3 g, 11 mol) was added dropwise to a stirred mixture of 19 g (0.091 mol) 2-nitro-4-(trifluoromethyl)benzonitrile and 100 mL of methanol at 20-30° C. After 1 hour at 25° C, 100 mL of water was added. The solids were collected on a filter and washed with water. The solids were dried to yield 12.5 g of product melting at 59-61° C.
$^1$H NMR ($CDCl_3$) δ 4.00 (s, 3H), 7.2-7.7 (3H).

Step C

2-Methoxy-4-(trifluoromethyl)benzaldehyde

Diisobutylaluminum hydride (1.5 M in toluene, 35 mL, 0.05 mol) was added over a 1 hour period to a mixture of 10.4 g (0.05 mol) 2-methoxy-4-(trifluoromethyl)benzonitrile and 50 mL toluene at -10° C. The mixture was agitated for 3 hours at 20-25° C and then poured into a mixture of 15 mL of concentrated hydrochloric acid and 50 mL of water at 0-5° C. The mixture was extracted with three 50 mL portions of toluene. The combined extracts were washed with water, dried over anhydrous magnesium sulfate, filtered, and evaporated. An oil (10.3 g) was obtained which, according to g-c analysis, consisted of about 90% of the aldehyde, 4% of unreacted nitrile and 6% of the benzylamine. The infrared spectrum exhibited a strong band at 5.9 μ and only a very weak band at 4.5 μ. Recrystallization from cyclohexanes yielded colorless

crystals melting at 54-55° C.
$^1$H NMR ($CDCl_3$) δ 4.00 (s, 3H), 7.22 (s, 1H), 7.30 (d, 1H), 7.92 (d, 1H), 10.49 (s, 1H).

Step D

2-Hydroxy-4-(trifluoromethyl)benzaldehyde

A mixture of 10.0 g (0.042 mol) 2-methoxy-4-(trifluoromethyl)benzaldehyde, 5.3 g (0.126 mol) lithium chloride, and 50 mL of dimethylformamide was heated at 155° C for 3 hours. The reaction mass was cooled, diluted with 100 mL of water, acidified with hydrochloric acid, and then extracted three times with 100 mL of ether. The ether extracts were washed with water, dried over anhydrous magnesium sulfate, and evaporated to provide 6.8 g of the product as an oil.
$^1$H NMR ($CDCl_3$) δ 7.01-7.88 (m, 4H), 11.16 (s, 1H).

Step E

Methyl 2-[[2-formyl-5-(trifluoromethyl)phenoxy]methyl]-2-propenoate

A mixture of 19.0 g (0.10 mol) 2-hydroxy-4-(trifluoromethyl)benzaldehyde, 13.8 g potassium carbonate, 19.6 g (0.11 mol) methyl(2-bromomethyl)-2-propenoate and 75 mL of dimethylformamide was heated at 50° C for 1 hour. After cooling to 20° C, 100 mL of water was added. The solids were collected on a filter, washed with water, and dried to yield 22.4 g of a colorless solid. The product was recrystallized from cyclohexane to yield a colorless solid melting at 77-79° C.
$^1$H NMR ($CDCl_3$) δ 3.84 (s, 3H), 4.92 (t, 2H), 6.06 (q, 1H), 6.50 (d, 1H), 7.26 (s, 1H), 7.32 (d, 1H), 7.96 (d, 1H), 10.53 (s, 1H).

Step F

Methyl 2-[[2-[[(diethoxyphosphinyl)hydrazono]methyl]-5-(trifluoromethyl)phenoxy]methyl]-2-propenoate

To a mixture of 14.4 g (0.050 mol) of the product of Step E and 50 mL of diethylether, 13.2 g (0.052 mol, assay 66%) of diethyl phosphorohydrazidate was added at 25° C. After agitating for 2 hours, the solids were collected on a filter and washed with 25 mL of ether, then with water. The solids were dried to yield 18.4 g of a colorless solid melting at 143-144° C.

Step G

Methyl 2-(diethoxyphosphinyl)-2,3,3a,4-tetrahydro-7-(trifluoromethyl)[1]benzopyrano[4,3-c]pyrazole-3a-carboxylate

To a mixture of 21.9 g (0.050 mol) of the product of Step F, 7.4 g (0.055 mol) of N-chlorosuccinimide and 750 mL of methylene chloride were added, and the mixture was heated to reflux. A mixture of 7.5 g (0.07 mol) triethylamine and 50 mL of methylene chloride was added over a 4 hour period. The reaction mass was refluxed for an additional 1 hour. After cooling to 25° C, the methylene chloride solution was washed three times with 150 mL of water. The methylene chloride solution was dried with magnesium sulfate, filtered, and the solvent was evaporated. An oil remained (24 g) which solidified upon standing. The

solid was recrystallized from a mixture of cyclohexanes and ethyl acetate to yield a colorless solid melting at 85-86° C.

Step H

Methyl 2,3,3a,4-tetrahydro-7-(trifluoromethyl)[1]benzopyrano[4,3-c]pyrazole-3a-carboxylate, hydrochloride

A mixture of the product of Step G (38 g, 0.087 mol), 200 mL of methanol, and 18 g (0.50 mol) hydrogen chloride was heated at reflux (66° C) for 2 1/2 hours. The solution was concentrated, toluene (50 mL) was added to the residue and, the mixture concentrated again. Another 50 mL toluene was added and evaporated a second time. After adding 100 mL of toluene, the slurry was chilled, and then filtered. The solid was washed with toluene, then hexanes to yield 26 g of a pale yellow product, m.p. 175-176° C, dec. Recrystallization from a mixture of tetrahydrofuran and methanol gave a colorless solid, m.p. 185-186° C, dec.

Step I

Methyl 2,3,3a,4-tetrahydro-7-(trifluoromethyl)-2-[[[4-(trifluoromethyl)phenyl]amino]carbonyl][1]benzopyrano[4,3-c]-pyrazole-3a-carboxylate

A mixture of 16.8 g of the product from Step H was stirred with 6.1 g of triethylamine and 100 mL of acetonitrile at 10-15° C and 10.8 g of α,α,α-trifluoro-p-tolyl isocyanate was added. The mixture was stirred for 2 hours at 20° C, water (100 mL) was added, and the precipitated product was filtered, washed with water and dried to provide 20.0 g of colorless product, m.p. 191-193° C. Recrystallization from THF/cyclohexane gave a product which sintered at 192-193° C, solidified, and remelted at 206-208° C.

The same product was also prepared by suspending 3.37 g of the product from Step H (83.6% purity) and 2.07 g of 4-(trifluoromethyl)phenylcarbamoyl fluoride in 18 mL of dichloromethane, adding dropwise 2.02 g of triethylamine, stirring for 15 min at ambient temperature, partitioning between ice-water and 25 mL of dichloromethane, washing the organic extract with water, drying with $MgSO_4$, and evaporating the solvent. The residue was washed with cold diethyl ether to provide 3.50 g (86% yield) of colorless product, m.p. 205.5-206° C (after initial melting at 191° C).

EXAMPLE 6

Methyl 7-trifluoromethyl-2,3,3a,4-tetrahydro-2-[[[4-(trifluoromethoxy)phenyl]amino]carbonyl][1]benzopyrano[4,3-c]-pyrazole-3-α-carboxylate

Application of the procedure described in Example 5 and substitution of 4-trifluoromethoxyphenylisocyanate afforded 0.163 g of a white solid, m.p. 161-163° C.
200 MHz $^1H$ NMR ($CDCl_3$) δ 3.77 (s, 3H), 3.85 (d, 1H), 4.22 (d, 1H), 4.52 (d, 1H), 5.08 (d, 1H), 7.2-7.35 (m, 4H), 7.52 (d, 2H), 7.98 (d, 1H), 8.0 (s, 1H).
IR (nujol) 3360, 1724, 1690 $cm^{-1}$.

By the general procedures described above, or obvious modifications thereof, the compounds of Tables 1 through 8 can be prepared.

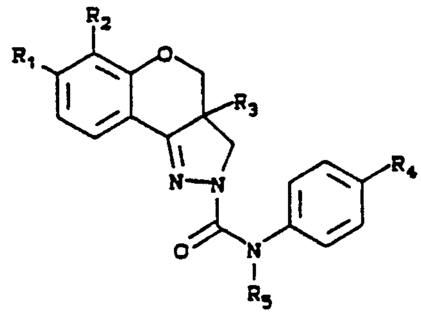

(R$_1$ is H)

TABLE 1

| $R_2$ | $R_3$ | $R_4$ | $R_5$ |
|---|---|---|---|
| F | Me | $CF_3$ | H |
| F | Et | $CF_3$ | H |
| F | nPr | $CF_3$ | H |
| F | iPr | $CF_3$ | H |
| F | nBu | $CF_3$ | H |
| F | sBu | $CF_3$ | H |
| F | iBu | $CF_3$ | H |
| F | tBu | $CF_3$ | H |
| F | 1-Pentyl | $CF_3$ | H |
| F | 2-Pentyl | $CF_3$ | H |
| F | cyclopropyl | $CF_3$ | H |
| F | cyclobutyl | $CF_3$ | H |
| F | cyclopentyl | $CF_3$ | H |
| F | cyclohexyl | $CF_3$ | H |
| F | $CO_2Me$ | $CF_3$ | H |
| F | Ph | $CF_3$ | H |
| F | 4-F-Ph | $CF_3$ | H |
| F | 4-Cl-Ph | $CF_3$ | H |
| F | Me | $OCF_3$ | H |
| F | Et | $OCF_3$ | H |
| F | nPr | $OCF_3$ | H |

| $R_2$ | $R_3$ | $R_4$ | $R_5$ |
|---|---|---|---|
| F | iPr | $OCF_3$ | H |
| F | nBu | $OCF_3$ | H |
| F | sBu | $OCF_3$ | H |
| F | iBu | $OCF_3$ | H |
| F | tBu | $OCF_3$ | H |
| F | $CO_2Me$ | $OCF_3$ | H |
| F | Ph | $OCF_3$ | H |
| F | 4-F-Ph | $OCF_3$ | H |
| F | 4-Cl-Ph | $OCF_3$ | H |
| F | Me | Cl | H |
| F | Et | Cl | H |
| F | nPr | Cl | H |
| F | iPr | Cl | H |
| F | nBu | Cl | H |
| F | sBu | Cl | H |
| F | iBu | Cl | H |
| F | tBu | Cl | H |
| F | 1-pentyl | Cl | H |
| F | 2-pentyl | Cl | H |
| F | cyclopropyl | Cl | H |
| F | cyclobutyl | Cl | H |
| F | cyclopentyl | Cl | H |
| F | cyclohexyl | Cl | H |
| F | $CO_2Me$ | Cl | H |
| F | Ph | Cl | H |
| F | 4-F-Ph | Cl | H |
| F | 4-Cl-Ph | Cl | H |
| F | Me | Br | H |
| F | Et | Br | H |
| F | nPr | Br | H |
| F | iPr | Br | H |
| F | nBu | Br | H |

| $R_2$ | $R_3$ | $R_4$ | $R_5$ |
|---|---|---|---|
| F | sBu | Br | H |
| F | iBu | Br | H |
| F | tBu | Br | H |
| F | 1-pentyl | Br | H |
| F | 2-pentyl | Br | H |
| F | cyclopropyl | Br | H |
| F | cyclobutyl | Br | H |
| F | cyclopentyl | Br | H |
| F | cyclohexyl | Br | H |
| F | $CO_2Me$ | Br | H |
| F | Ph | Br | H |
| F | 4-F-Ph | Br | H |
| F | 4-Cl-Ph | Br | H |
| F | Me | $CF_3$ | $COCH_3$ |
| F | Et | $CF_3$ | $COCH_3$ |
| F | nPr | $CF_3$ | $COCH_3$ |
| F | iPr | $CF_3$ | $COCH_3$ |
| F | nBu | $CF_3$ | $COCH_3$ |
| F | sBu | $CF_3$ | $COCH_3$ |
| F | iBu | $CF_3$ | $COCH_3$ |
| F | tBu | $CF_3$ | $COCH_3$ |
| F | $CO_2Me$ | $CF_3$ | $COCH_3$ |
| F | Ph | $CF_3$ | $COCH_3$ |
| F | 4-F-Ph | $CF_3$ | $COCH_3$ |
| F | 4-Cl-Ph | $CF_3$ | $COCH_3$ |
| F | Me | $OCF_3$ | $COCH_3$ |
| F | Et | $OCF_3$ | $COCH_3$ |
| F | nPr | $OCF_3$ | $COCH_3$ |
| F | iPr | $OCF_3$ | $COCH_3$ |
| F | nBu | $OCF_3$ | $COCH_3$ |
| F | sBu | $OCF_3$ | $COCH_3$ |
| F | iBu | $OCF_3$ | $COCH_3$ |

| $R_2$ | $R_3$ | $R_4$ | $R_5$ |
|---|---|---|---|
| F | tBu | $OCF_3$ | $COCH_3$ |
| F | $CO_2Me$ | $OCF_3$ | $COCH_3$ |
| F | Ph | $OCF_3$ | $COCH_3$ |
| F | 4-F-Ph | $OCF_3$ | $COCH_3$ |
| F | 4-Cl-Ph | $OCF_3$ | $COCH_3$ |
| F | Me | Cl | $COCH_3$ |
| F | Et | Cl | $COCH_3$ |
| F | nPr | Cl | $COCH_3$ |
| F | iPr | Cl | $COCH_3$ |
| F | nBu | Cl | $COCH_3$ |
| F | sBu | Cl | $COCH_3$ |
| F | iBu | Cl | $COCH_3$ |
| F | tBu | Cl | $COCH_3$ |
| F | $CO_2Me$ | Cl | $COCH_3$ |
| F | Ph | Cl | $COCH_3$ |
| F | 4-F-Ph | Cl | $COCH_3$ |
| F | 4-Cl-Ph | Cl | $COCH_3$ |
| F | Me | Br | $COCH_3$ |
| F | Et | Br | $COCH_3$ |
| F | nPr | Br | $COCH_3$ |
| F | iPr | Br | $COCH_3$ |
| F | nBu | Br | $COCH_3$ |
| F | sBu | Br | $COCH_3$ |
| F | iBu | Br | $COCH_3$ |
| F | tBu | Br | $COCH_3$ |
| F | $CO_2Me$ | Br | $COCH_3$ |
| F | Ph | Br | $COCH_3$ |
| F | 4-F-Ph | Br | $COCH_3$ |
| F | 4-Cl-Ph | Br | $COCH_3$ |
| F | Me | $CF_3$ | $CO_2CH_3$ |
| F | Et | $CF_3$ | $CO_2CH_3$ |
| F | nPr | $CF_3$ | $CO_2CH_3$ |

| $R_2$ | $R_3$ | $R_4$ | $R_5$ |
|---|---|---|---|
| F | iPr | $CF_3$ | $CO_2CH_3$ |
| F | nBu | $CF_3$ | $CO_2CH_3$ |
| F | sBu | $CF_3$ | $CO_2CH_3$ |
| F | iBu | $CF_3$ | $CO_2CH_3$ |
| F | tBu | $CF_3$ | $CO_2CH_3$ |
| F | $CO_2Me$ | $CF_3$ | $CO_2CH_3$ |
| F | Ph | $CF_3$ | $CO_2CH_3$ |
| F | 4-F-Ph | $CF_3$ | $CO_2CH_3$ |
| F | 4-Cl-Ph | $CF_3$ | $CO_2CH_3$ |
| F | Me | $OCF_3$ | $CO_2CH_3$ |
| F | Et | $OCF_3$ | $CO_2CH_3$ |
| F | nPr | $OCF_3$ | $CO_2CH_3$ |
| F | iPr | $OCF_3$ | $CO_2CH_3$ |
| F | nBu | $OCF_3$ | $CO_2CH_3$ |
| F | sBu | $OCF_3$ | $CO_2CH_3$ |
| F | iBu | $OCF_3$ | $CO_2CH_3$ |
| F | tBu | $OCF_3$ | $CO_2CH_3$ |
| F | $CO_2Me$ | $OCF_3$ | $CO_2CH_3$ |
| F | Ph | $OCF_3$ | $CO_2CH_3$ |
| F | 4-F-Ph | $OCF_3$ | $CO_2CH_3$ |
| F | 4-Cl-Ph | $OCF_3$ | $CO_2CH_3$ |
| F | Me | Cl | $CO_2CH_3$ |
| F | Et | Cl | $CO_2CH_3$ |
| F | nPr | Cl | $CO_2CH_3$ |
| F | iPr | Cl | $CO_2CH_3$ |
| F | nBu | Cl | $CO_2CH_3$ |
| F | sBu | Cl | $CO_2CH_3$ |
| F | iBu | Cl | $CO_2CH_3$ |
| F | tBu | Cl | $CO_2CH_3$ |
| F | $CO_2Me$ | Cl | $CO_2CH_3$ |
| F | Ph | Cl | $CO_2CH_3$ |
| F | 4-F-Ph | Cl | $CO_2CH_3$ |
| F | 4-Cl-Ph | Cl | $CO_2CH_3$ |

| $R_2$ | $R_3$ | $R_4$ | $R_5$ |
|---|---|---|---|
| F | Me | Br | $CO_2CH_3$ |
| F | Et | Br | $CO_2CH_3$ |
| F | nPr | Br | $CO_2CH_3$ |
| F | iPr | Br | $CO_2CH_3$ |
| F | nBu | Br | $CO_2CH_3$ |
| F | sBu | Br | $CO_2CH_3$ |
| F | iBu | Br | $CO_2CH_3$ |
| F | tBu | Br | $CO_2CH_3$ |
| F | $CO_2Me$ | Br | $CO_2CH_3$ |
| F | Ph | Br | $CO_2CH_3$ |
| F | 4-F-Ph | Br | $CO_2CH_3$ |
| F | 4-Cl-Ph | Br | $CO_2CH_3$ |

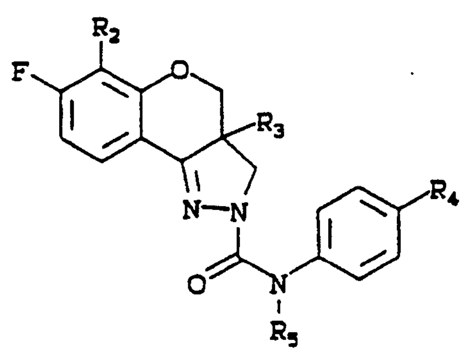

TABLE 2

| $R_2$ | $R_3$ | $R_4$ | $R_5$ |
|---|---|---|---|
| H | Me | $CF_3$ | H |
| H | Et | $CF_3$ | H |
| H | nPr | $CF_3$ | H |
| H | iPr | $CF_3$ | H |
| H | nBu | $CF_3$ | H |
| H | sBu | $CF_3$ | H |
| H | iBu | $CF_3$ | H |
| H | tBu | $CF_3$ | H |
| H | 1-pentyl | $CF_3$ | H |
| H | 2-pentyl | $CF_3$ | H |
| H | cyclopropyl | $CF_3$ | H |
| H | cyclobutyl | $CF_3$ | H |
| H | cyclopentyl | $CF_3$ | H |
| H | cyclohexyl | $CF_3$ | H |
| H | $CO_2Me$ | $CF_3$ | H |
| H | Ph | $CF_3$ | H |
| H | 4-F-Ph | $CF_3$ | H |
| H | 4-Cl-Ph | $CF_3$ | H |
| H | Me | $OCF_3$ | H |
| H | Et | $OCF_3$ | H |
| H | nPr | $OCF_3$ | H |

| $R_2$ | $R_3$ | $R_4$ | $R_5$ |
|---|---|---|---|
| H | iPr | $OCF_3$ | H |
| H | nBu | $OCF_3$ | H |
| H | sBu | $OCF_3$ | H |
| H | iBu | $OCF_3$ | H |
| H | tBu | $OCF_3$ | H |
| H | $CO_2Me$ | $OCF_3$ | H |
| H | Ph | $OCF_3$ | H |
| H | 4-F-Ph | $OCF_3$ | H |
| H | 4-Cl-Ph | $OCF_3$ | H |
| H | Me | Cl | H |
| H | Et | Cl | H |
| H | nPr | Cl | H |
| H | iPr | Cl | H |
| H | nBu | Cl | H |
| H | sBu | Cl | H |
| H | iBu | Cl | H |
| H | tBu | Cl | H |
| H | 1-pentyl | Cl | H |
| H | 2-pentyl | Cl | H |
| H | cyclopropyl | Cl | H |
| H | cyclobutyl | Cl | H |
| H | cyclopentyl | Cl | H |
| H | cyclohexyl | Cl | H |
| H | $CO_2Me$ | Cl | H |
| H | Ph | Cl | H |
| H | 4-F-Ph | Cl | H |
| H | 4-Cl-Ph | Cl | H |
| H | Me | Br | H |
| H | Et | Br | H |
| H | nPr | Br | H |
| H | iPr | Br | H |
| H | nBu | Br | H |

| $R_2$ | $R_3$ | $R_4$ | $R_5$ |
|---|---|---|---|
| H | sBu | Br | H |
| H | iBu | Br | H |
| H | tBu | Br | H |
| H | 1-pentyl | Br | H |
| H | 2-pentyl | Br | H |
| H | cyclopropyl | Br | H |
| H | cyclobutyl | Br | H |
| H | cyclopentyl | Br | H |
| H | cyclohexyl | Br | H |
| H | $CO_2Me$ | Br | H |
| H | Ph | Br | H |
| H | 4-F-Ph | Br | H |
| H | 4-Cl-Ph | Br | H |
| H | Me | $CF_3$ | $COCH_3$ |
| H | Et | $CF_3$ | $COCH_3$ |
| H | nPr | $CF_3$ | $COCH_3$ |
| H | iPr | $CF_3$ | $COCH_3$ |
| H | nBu | $CF_3$ | $COCH_3$ |
| H | sBu | $CF_3$ | $COCH_3$ |
| H | iBu | $CF_3$ | $COCH_3$ |
| H | tBu | $CF_3$ | $COCH_3$ |
| H | $CO_2Me$ | $CF_3$ | $COCH_3$ |
| H | Ph | $CF_3$ | $COCH_3$ |
| H | 4-F-Ph | $CF_3$ | $COCH_3$ |
| H | 4-Cl-Ph | $CF_3$ | $COCH_3$ |
| H | Me | $OCF_3$ | $COCH_3$ |
| H | Et | $OCF_3$ | $COCH_3$ |
| H | nPr | $OCF_3$ | $COCH_3$ |
| H | iPr | $OCF_3$ | $COCH_3$ |
| H | nBu | $OCF_3$ | $COCH_3$ |
| H | sBu | $OCF_3$ | $COCH_3$ |
| H | iBu | $OCF_3$ | $COCH_3$ |

| $R_2$ | $R_3$ | $R_4$ | $R_5$ |
|---|---|---|---|
| H | tBu | $OCF_3$ | $COCH_3$ |
| H | $CO_2Me$ | $OCF_3$ | $COCH_3$ |
| H | Ph | $OCF_3$ | $COCH_3$ |
| H | 4-F-Ph | $OCF_3$ | $COCH_3$ |
| H | 4-Cl-Ph | $OCF_3$ | $COCH_3$ |
| H | Me | Cl | $COCH_3$ |
| H | Et | Cl | $COCH_3$ |
| H | nPr | Cl | $COCH_3$ |
| H | iPr | Cl | $COCH_3$ |
| H | nBu | Cl | $COCH_3$ |
| H | sBu | Cl | $COCH_3$ |
| H | iBu | Cl | $COCH_3$ |
| H | tBu | Cl | $COCH_3$ |
| H | $CO_2Me$ | Cl | $COCH_3$ |
| H | Ph | Cl | $COCH_3$ |
| H | 4-F-Ph | Cl | $COCH_3$ |
| H | 4-Cl-Ph | Cl | $COCH_3$ |
| H | Me | Br | $COCH_3$ |
| H | Et | Br | $COCH_3$ |
| H | nPr | Br | $COCH_3$ |
| H | iPr | Br | $COCH_3$ |
| H | nBu | Br | $COCH_3$ |
| H | sBu | Br | $COCH_3$ |
| H | iBu | Br | $COCH_3$ |
| H | tBu | Br | $COCH_3$ |
| H | $CO_2Me$ | Br | $COCH_3$ |
| H | Ph | Br | $COCH_3$ |
| H | 4-F-Ph | Br | $COCH_3$ |
| H | 4-Cl-Ph | Br | $COCH_3$ |
| H | Me | $CF_3$ | $CO_2CH_3$ |
| H | Et | $CF_3$ | $CO_2CH_3$ |
| H | nPr | $CF_3$ | $CO_2CH_3$ |

| $R_2$ | $R_3$ | $R_4$ | $R_5$ |
|---|---|---|---|
| H | iPr | $CF_3$ | $CO_2CH_3$ |
| H | nBu | $CF_3$ | $CO_2CH_3$ |
| H | sBu | $CF_3$ | $CO_2CH_3$ |
| H | iBu | $CF_3$ | $CO_2CH_3$ |
| H | tBu | $CF_3$ | $CO_2CH_3$ |
| H | $CO_2Me$ | $CF_3$ | $CO_2CH_3$ |
| H | Ph | $CF_3$ | $CO_2CH_3$ |
| H | 4-F-Ph | $CF_3$ | $CO_2CH_3$ |
| H | 4-Cl-Ph | $CF_3$ | $CO_2CH_3$ |
| H | Me | $OCF_3$ | $CO_2CH_3$ |
| H | Et | $OCF_3$ | $CO_2CH_3$ |
| H | nPr | $OCF_3$ | $CO_2CH_3$ |
| H | iPr | $OCF_3$ | $CO_2CH_3$ |
| H | nBu | $OCF_3$ | $CO_2CH_3$ |
| H | sBu | $OCF_3$ | $CO_2CH_3$ |
| H | iBu | $OCF_3$ | $CO_2CH_3$ |
| H | tBu | $OCF_3$ | $CO_2CH_3$ |
| H | $CO_2Me$ | $OCF_3$ | $CO_2CH_3$ |
| H | Ph | $OCF_3$ | $CO_2CH_3$ |
| H | 4-F-Ph | $OCF_3$ | $CO_2CH_3$ |
| H | 4-Cl-Ph | $OCF_3$ | $CO_2CH_3$ |
| H | Me | Cl | $CO_2CH_3$ |
| H | Et | Cl | $CO_2CH_3$ |
| H | nPr | Cl | $CO_2CH_3$ |
| H | iPr | Cl | $CO_2CH_3$ |
| H | nBu | Cl | $CO_2CH_3$ |
| H | sBu | Cl | $CO_2CH_3$ |
| H | iBu | Cl | $CO_2CH_3$ |
| H | tBu | Cl | $CO_2CH_3$ |
| H | $CO_2Me$ | Cl | $CO_2CH_3$ |
| H | Ph | Cl | $CO_2CH_3$ |
| H | 4-F-Ph | Cl | $CO_2CH_3$ |

| $R_2$ | $R_3$ | $R_4$ | $R_5$ |
|---|---|---|---|
| H | 4-Cl-Ph | Cl | $CO_2CH_3$ |
| H | Me | Br | $CO_2CH_3$ |
| H | Et | Br | $CO_2CH_3$ |
| H | nPr | Br | $CO_2CH_3$ |
| H | iPr | Br | $CO_2CH_3$ |
| H | nBu | Br | $CO_2CH_3$ |
| H | sBu | Br | $CO_2CH_3$ |
| H | iBu | Br | $CO_2CH_3$ |
| H | tBu | Br | $CO_2CH_3$ |
| H | $CO_2Me$ | Br | $CO_2CH_3$ |
| H | Ph | Br | $CO_2CH_3$ |
| H | 4-F-Ph | Br | $CO_2CH_3$ |
| H | 4-Cl-Ph | Br | $CO_2CH_3$ |

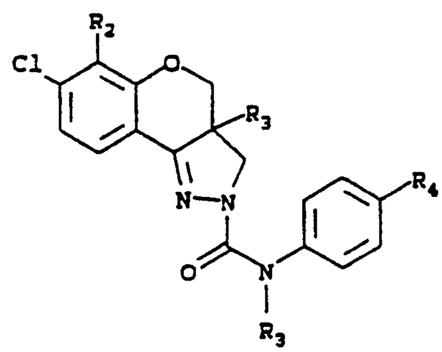

TABLE 3

| $R_2$ | $R_3$ | $R_4$ | $R_5$ |
|---|---|---|---|
| H | Me | $CF_3$ | H |
| H | Et | $CF_3$ | H |
| H | nPr | $CF_3$ | H |
| H | iPr | $CF_3$ | H |
| H | nBu | $CF_3$ | H |
| H | sBu | $CF_3$ | H |
| H | iBu | $CF_3$ | H |
| H | tBu | $CF_3$ | H |
| H | 1-pentyl | $CF_3$ | H |
| H | 2-pentyl | $CF_3$ | H |
| H | cyclopropyl | $CF_3$ | H |
| H | cyclobutyl | $CF_3$ | H |
| H | cyclopentyl | $CF_3$ | H |
| H | cyclohexyl | $CF_3$ | H |
| H | $CO_2Me$ | $CF_3$ | H |
| H | Ph | $CF_3$ | H |
| H | 4-F-Ph | $CF_3$ | H |
| H | 4-Cl-Ph | $CF_3$ | H |
| H | Me | $OCF_3$ | H |
| H | Et | $OCF_3$ | H |

| $R_2$ | $R_3$ | $R_4$ | $R_5$ |
|---|---|---|---|
| H | nPr | $OCF_3$ | H |
| H | iPr | $OCF_3$ | H |
| H | nBu | $OCF_3$ | H |
| H | sBu | $OCF_3$ | H |
| H | iBu | $OCF_3$ | H |
| H | tBu | $OCF_3$ | H |
| H | $CO_2Me$ | $OCF_3$ | H |
| H | Ph | $OCF_3$ | H |
| H | 4-F-Ph | $OCF_3$ | H |
| H | 4-Cl-Ph | $OCF_3$ | H |
| H | Me | Cl | H |
| H | Et | Cl | H |
| H | nPr | Cl | H |
| H | iPr | Cl | H |
| H | nBu | Cl | H |
| H | sBu | Cl | H |
| H | iBu | Cl | H |
| H | tBu | Cl | H |
| H | 1-pentyl | Cl | H |
| H | 2-pentyl | Cl | H |
| H | cyclopropyl | Cl | H |
| H | cyclobutyl | Cl | H |
| H | cyclopentyl | Cl | H |
| H | cyclohexyl | Cl | H |
| H | $CO_2Me$ | Cl | H |
| H | Ph | Cl | H |
| H | 4-F-Ph | Cl | H |
| H | 4-Cl-Ph | Cl | H |
| H | Me | Br | H |
| H | Et | Br | H |
| H | nPr | Br | H |
| H | iPr | Br | H |
| H | nBu | Br | H |

| $R_2$ | $R_3$ | $R_4$ | $R_5$ |
|---|---|---|---|
| H | sBu | Br | H |
| H | iBu | Br | H |
| H | tBu | Br | H |
| H | 1-pentyl | Br | H |
| H | 2-pentyl | Br | H |
| H | cyclopropyl | Br | H |
| H | cyclobutyl | Br | H |
| H | cyclopentyl | Br | H |
| H | cyclohexyl | Br | H |
| H | $CO_2Me$ | Br | H |
| H | Ph | Br | H |
| H | 4-F-Ph | Br | H |
| H | 4-Cl-Ph | Br | H |
| H | Me | $CF_3$ | $COCH_3$ |
| H | Et | $CF_3$ | $COCH_3$ |
| H | nPr | $CF_3$ | $COCH_3$ |
| H | iPr | $CF_3$ | $COCH_3$ |
| H | nBu | $CF_3$ | $COCH_3$ |
| H | sBu | $CF_3$ | $COCH_3$ |
| H | iBu | $CF_3$ | $COCH_3$ |
| H | tBu | $CF_3$ | $COCH_3$ |
| H | 1-pentyl | $CF_3$ | $COCH_3$ |
| H | 2-pentyl | $CF_3$ | $COCH_3$ |
| H | cyclopropyl | $CF_3$ | $COCH_3$ |
| H | cyclobutyl | $CF_3$ | $COCH_3$ |
| H | cyclopentyl | $CF_3$ | $COCH_3$ |
| H | cyclohexyl | $CF_3$ | $COCH_3$ |
| H | $CO_2Me$ | $CF_3$ | $COCH_3$ |
| H | Ph | $CF_3$ | $COCH_3$ |
| H | 4-F-Ph | $CF_3$ | $COCH_3$ |
| H | 4-Cl-Ph | $CF_3$ | $COCH_3$ |
| H | Me | $OCF_3$ | $COCH_3$ |

| $R_2$ | $R_3$ | $R_4$ | $R_5$ |
|---|---|---|---|
| H | Et | $OCF_3$ | $COCH_3$ |
| H | nPr | $OCF_3$ | $COCH_3$ |
| H | iPr | $OCF_3$ | $COCH_3$ |
| H | nBu | $OCF_3$ | $COCH_3$ |
| H | sBu | $OCF_3$ | $COCH_3$ |
| H | iBu | $OCF_3$ | $COCH_3$ |
| H | tBu | $OCF_3$ | $COCH_3$ |
| H | $CO_2Me$ | $OCF_3$ | $COCH_3$ |
| H | Ph | $OCF_3$ | $COCH_3$ |
| H | 4-F-Ph | $OCF_3$ | $COCH_3$ |
| H | 4-Cl-Ph | $OCF_3$ | $COCH_3$ |
| H | Me | Cl | $COCH_3$ |
| H | Et | Cl | $COCH_3$ |
| H | nPr | Cl | $COCH_3$ |
| H | iPr | Cl | $COCH_3$ |
| H | nBu | Cl | $COCH_3$ |
| H | sBu | Cl | $COCH_3$ |
| H | iBu | Cl | $COCH_3$ |
| H | tBu | Cl | $COCH_3$ |
| H | 1-pentyl | Cl | $COCH_3$ |
| H | 2-pentyl | Cl | $COCH_3$ |
| H | cyclopropyl | Cl | $COCH_3$ |
| H | cyclobutyl | Cl | $COCH_3$ |
| H | cyclopentyl | Cl | $COCH_3$ |
| H | cyclohexyl | Cl | $COCH_3$ |
| H | $CO_2Me$ | Cl | $COCH_3$ |
| H | Ph | Cl | $COCH_3$ |
| H | 4-F-Ph | Cl | $COCH_3$ |
| H | 4-Cl-Ph | Cl | $COCH_3$ |
| H | Me | Br | $COCH_3$ |
| H | Et | Br | $COCH_3$ |
| H | nPr | Br | $COCH_3$ |

| $R_2$ | $R_3$ | $R_4$ | $R_5$ |
|---|---|---|---|
| H | iPr | Br | $COCH_3$ |
| H | nBu | Br | $COCH_3$ |
| H | sBu | Br | $COCH_3$ |
| H | iBu | Br | $COCH_3$ |
| H | tBu | Br | $COCH_3$ |
| H | 1-pentyl | Br | $COCH_3$ |
| H | 2-pentyl | Br | $COCH_3$ |
| H | cyclopropyl | Br | $COCH_3$ |
| H | cyclobutyl | Br | $COCH_3$ |
| H | cyclopentyl | Br | $COCH_3$ |
| H | cyclohexyl | Br | $COCH_3$ |
| H | $CO_2Me$ | Br | $COCH_3$ |
| H | Ph | Br | $COCH_3$ |
| H | 4-F-Ph | Br | $COCH_3$ |
| H | 4-Cl-Ph | Br | $COCH_3$ |
| H | Me | $CF_3$ | $CO_2CH_3$ |
| H | Et | $CF_3$ | $CO_2CH_3$ |
| H | nPr | $CF_3$ | $CO_2CH_3$ |
| H | iPr | $CF_3$ | $CO_2CH_3$ |
| H | nBu | $CF_3$ | $CO_2CH_3$ |
| H | sBu | $CF_3$ | $CO_2CH_3$ |
| H | iBu | $CF_3$ | $CO_2CH_3$ |
| H | tBu | $CF_3$ | $CO_2CH_3$ |
| H | 1-pentyl | $CF_3$ | $CO_2CH_3$ |
| H | 2-pentyl | $CF_3$ | $CO_2CH_3$ |
| H | cyclopropyl | $CF_3$ | $CO_2CH_3$ |
| H | cyclobutyl | $CF_3$ | $CO_2CH_3$ |
| H | cyclopentyl | $CF_3$ | $CO_2CH_3$ |
| H | cyclohexyl | $CF_3$ | $CO_2CH_3$ |
| H | $CO_2Me$ | $CF_3$ | $CO_2CH_3$ |
| H | Ph | $CF_3$ | $CO_2CH_3$ |
| H | 4-F-Ph | $CF_3$ | $CO_2CH_3$ |

| $R_2$ | $R_3$ | $R_4$ | $R_5$ |
|---|---|---|---|
| H | 4-Cl-Ph | $CF_3$ | $CO_2CH_3$ |
| H | Me | $OCF_3$ | $CO_2CH_3$ |
| H | Et | $OCF_3$ | $CO_2CH_3$ |
| H | nPr | $OCF_3$ | $CO_2CH_3$ |
| H | iPr | $OCF_3$ | $CO_2CH_3$ |
| H | nBu | $OCF_3$ | $CO_2CH_3$ |
| H | sBu | $OCF_3$ | $CO_2CH_3$ |
| H | iBu | $OCF_3$ | $CO_2CH_3$ |
| H | tBu | $OCF_3$ | $CO_2CH_3$ |
| H | $CO_2Me$ | $OCF_3$ | $CO_2CH_3$ |
| H | Ph | $OCF_3$ | $CO_2CH_3$ |
| H | 4-F-Ph | $OCF_3$ | $CO_2CH_3$ |
| H | 4-Cl-Ph | $OCF_3$ | $CO_2CH_3$ |
| H | Me | Cl | $CO_2CH_3$ |
| H | Et | Cl | $CO_2CH_3$ |
| H | nPr | Cl | $CO_2CH_3$ |
| H | iPr | Cl | $CO_2CH_3$ |
| H | nBu | Cl | $CO_2CH_3$ |
| H | sBu | Cl | $CO_2CH_3$ |
| H | iBu | Cl | $CO_2CH_3$ |
| H | tBu | Cl | $CO_2CH_3$ |
| H | 1-pentyl | Cl | $CO_2CH_3$ |
| H | 2-pentyl | Cl | $CO_2CH_3$ |
| H | cyclopropyl | Cl | $CO_2CH_3$ |
| H | cyclobutyl | Cl | $CO_2CH_3$ |
| H | cyclopentyl | Cl | $CO_2CH_3$ |
| H | cyclohexyl | Cl | $CO_2CH_3$ |
| H | $CO_2Me$ | Cl | $CO_2CH_3$ |
| H | Ph | Cl | $CO_2CH_3$ |
| H | 4-F-Ph | Cl | $CO_2CH_3$ |
| H | 4-Cl-Ph | Cl | $CO_2CH_3$ |
| H | Me | Br | $CO_2CH_3$ |

| $R_2$ | $R_3$ | $R_4$ | $R_5$ |
|---|---|---|---|
| H | Et | Br | $CO_2CH_3$ |
| H | nPr | Br | $CO_2CH_3$ |
| H | iPr | Br | $CO_2CH_3$ |
| H | nBu | Br | $CO_2CH_3$ |
| H | sBu | Br | $CO_2CH_3$ |
| H | iBu | Br | $CO_2CH_3$ |
| H | tBu | Br | $CO_2CH_3$ |
| H | 1-pentyl | Br | $CO_2CH_3$ |
| H | 2-pentyl | Br | $CO_2CH_3$ |
| H | cyclopropyl | Br | $CO_2CH_3$ |
| H | cyclobutyl | Br | $CO_2CH_3$ |
| H | cyclopentyl | Br | $CO_2CH_3$ |
| H | cyclohexyl | Br | $CO_2CH_3$ |
| H | $CO_2Me$ | Br | $CO_2CH_3$ |
| H | Ph | Br | $CO_2CH_3$ |
| H | 4-F-Ph | Br | $CO_2CH_3$ |
| H | 4-Cl-Ph | Br | $CO_2CH_3$ |

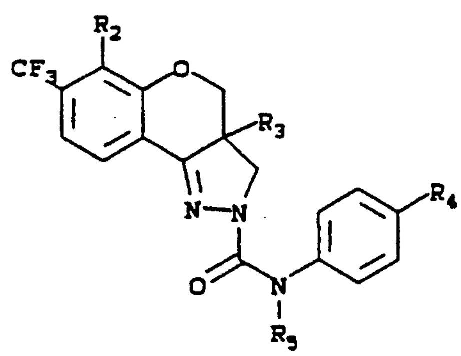

TABLE 4

| $R_2$ | $R_3$ | $R_4$ | $R_5$ |
|---|---|---|---|
| H | Me | $CF_3$ | H |
| H | Et | $CF_3$ | H |
| H | nPr | $CF_3$ | H |
| H | iPr | $CF_3$ | H |
| H | nBu | $CF_3$ | H |
| H | sBu | $CF_3$ | H |
| H | iBu | $CF_3$ | H |
| H | tBu | $CF_3$ | H |
| H | 1-pentyl | CF3 | H |
| H | 2-pentyl | CF3 | H |
| H | cyclopropyl | CF3 | H |
| H | cyclobutyl | CF3 | H |
| H | cyclopentyl | CF3 | H |
| H | cyclohexyl | CF3 | H |
| H | $CO_2Me$ | $CF_3$ | H |
| H | Ph | $CF_3$ | H |
| H | 4-F-Ph | $CF_3$ | H |
| H | 4-Cl-Ph | $CF_3$ | H |
| H | Me | $OCF_3$ | H |
| H | Et | $OCF_3$ | H |

| $R_2$ | $R_3$ | $R_4$ | $R_5$ |
|---|---|---|---|
| H | nPr | $OCF_3$ | H |
| H | iPr | $OCF_3$ | H |
| H | nBu | $OCF_3$ | H |
| H | sBu | $OCF_3$ | H |
| H | iBu | $OCF_3$ | H |
| H | tBu | $OCF_3$ | H |
| H | $CO_2Me$ | $OCF_3$ | H |
| H | Ph | $OCF_3$ | H |
| H | 4-F-Ph | $OCF_3$ | H |
| H | 4-Cl-Ph | $OCF_3$ | H |
| H | Me | Cl | H |
| H | Et | Cl | H |
| H | nPr | Cl | H |
| H | iPr | Cl | H |
| H | nBu | Cl | H |
| H | sBu | Cl | H |
| H | iBu | Cl | H |
| H | tBu | Cl | H |
| H | 1-pentyl | Cl | H |
| H | 2-pentyl | Cl | H |
| H | cyclopropyl | Cl | H |
| H | cyclobutyl | Cl | H |
| H | cyclopentyl | Cl | H |
| H | cyclohexyl | Cl | H |
| H | $CO_2Me$ | Cl | H |
| H | Ph | Cl | H |
| H | 4-F-Ph | Cl | H |
| H | 4-Cl-Ph | Cl | H |
| H | Me | Br | H |
| H | Et | Br | H |
| H | nPr | Br | H |
| H | iPr | Br | H |

| $R_2$ | $R_3$ | $R_4$ | $R_5$ |
|---|---|---|---|
| H | nBu | Br | H |
| H | sBu | Br | H |
| H | iBu | Br | H |
| H | 1-pentyl | Br | H |
| H | 2-pentyl | Br | H |
| H | cyclopropyl | Br | H |
| H | cyclobutyl | Br | H |
| H | cyclopentyl | Br | H |
| H | cyclohexyl | Br | H |
| H | tBu | Br | H |
| H | $CO_2Me$ | Br | H |
| H | Ph | Br | H |
| H | 4-F-Ph | Br | H |
| H | 4-Cl-Ph | Br | H |
| H | Me | $CF_3$ | $COCH_3$ |
| H | Et | $CF_3$ | $COCH_3$ |
| H | nPr | $CF_3$ | $COCH_3$ |
| H | iPr | $CF_3$ | $COCH_3$ |
| H | nBu | $CF_3$ | $COCH_3$ |
| H | sBu | $CF_3$ | $COCH_3$ |
| H | iBu | $CF_3$ | $COCH_3$ |
| H | tBu | $CF_3$ | $COCH_3$ |
| H | 1-pentyl | $CF_3$ | $COCH_3$ |
| H | 2-pentyl | $CF_3$ | $COCH_3$ |
| H | cyclopropyl | $CF_3$ | $COCH_3$ |
| H | cyclobutyl | $CF_3$ | $COCH_3$ |
| H | cyclopentyl | $CF_3$ | $COCH_3$ |
| H | cyclohexyl | $CF_3$ | $COCH_3$ |
| H | $CO_2Me$ | $CF_3$ | $COCH_3$ |
| H | Ph | $CF_3$ | $COCH_3$ |
| H | 4-F-Ph | $CF_3$ | $COCH_3$ |
| H | 4-Cl-Ph | $CF_3$ | $COCH_3$ |

| $R_2$ | $R_3$ | $R_4$ | $R_5$ |
|---|---|---|---|
| H | Me | $OCF_3$ | $COCH_3$ |
| H | Et | $OCF_3$ | $COCH_3$ |
| H | nPr | $OCF_3$ | $COCH_3$ |
| H | iPr | $OCF_3$ | $COCH_3$ |
| H | nBu | $OCF_3$ | $COCH_3$ |
| H | sBu | $OCF_3$ | $COCH_3$ |
| H | iBu | $OCF_3$ | $COCH_3$ |
| H | tBu | $OCF_3$ | $COCH_3$ |
| H | $CO_2Me$ | $OCF_3$ | $COCH_3$ |
| H | Ph | $OCF_3$ | $COCH_3$ |
| H | 4-F-Ph | $OCF_3$ | $COCH_3$ |
| H | 4-Cl-Ph | $OCF_3$ | $COCH_3$ |
| H | Me | Cl | $COCH_3$ |
| H | Et | Cl | $COCH_3$ |
| H | nPr | Cl | $COCH_3$ |
| H | iPr | Cl | $COCH_3$ |
| H | nBu | Cl | $COCH_3$ |
| H | sBu | Cl | $COCH_3$ |
| H | iBu | Cl | $COCH_3$ |
| H | tBu | Cl | $COCH_3$ |
| H | 1-pentyl | Cl | $COCH_3$ |
| H | 2-pentyl | Cl | $COCH_3$ |
| H | cyclopropyl | Cl | $COCH_3$ |
| H | cyclobutyl | Cl | $COCH_3$ |
| H | cyclopentyl | Cl | $COCH_3$ |
| H | cyclohexyl | Cl | $COCH_3$ |
| H | $CO_2Me$ | Cl | $COCH_3$ |
| H | Ph | Cl | $COCH_3$ |
| H | 4-F-Ph | Cl | $COCH_3$ |
| H | 4-Cl-Ph | Cl | $COCH_3$ |
| H | Me | Br | $COCH_3$ |
| H | Et | Br | $COCH_3$ |

| $R_2$ | $R_3$ | $R_4$ | $R_5$ |
|---|---|---|---|
| H | nPr | Br | $COCH_3$ |
| H | iPr | Br | $COCH_3$ |
| H | nBu | Br | $COCH_3$ |
| H | sBu | Br | $COCH_3$ |
| H | iBu | Br | $COCH_3$ |
| H | tBu | Br | $COCH_3$ |
| H | 1-pentyl | Br | $COCH_3$ |
| H | 2-pentyl | Br | $COCH_3$ |
| H | cyclopropyl | Br | $COCH_3$ |
| H | cyclobutyl | Br | $COCH_3$ |
| H | cyclopentyl | Br | $COCH_3$ |
| H | cyclohexyl | Br | $COCH_3$ |
| H | $CO_2Me$ | Br | $COCH_3$ |
| H | Ph | Br | $COCH_3$ |
| H | 4-F-Ph | Br | $COCH_3$ |
| H | 4-Cl-Ph | Br | $COCH_3$ |
| H | Me | $CF_3$ | $CO_2CH_3$ |
| H | Et | $CF_3$ | $CO_2CH_3$ |
| H | nPr | $CF_3$ | $CO_2CH_3$ |
| H | iPr | $CF_3$ | $CO_2CH_3$ |
| H | nBu | $CF_3$ | $CO_2CH_3$ |
| H | sBu | $CF_3$ | $CO_2CH_3$ |
| H | iBu | $CF_3$ | $CO_2CH_3$ |
| H | tBu | $CF_3$ | $CO_2CH_3$ |
| H | 1-pentyl | $CF_3$ | $CO_2CH_3$ |
| H | 2-pentyl | $CF_3$ | $CO_2CH_3$ |
| H | cyclopropyl | $CF_3$ | $CO_2CH_3$ |
| H | cyclobutyl | $CF_3$ | $CO_2CH_3$ |
| H | cyclopentyl | $CF_3$ | $CO_2CH_3$ |
| H | cyclohexyl | $CF_3$ | $CO_2CH_3$ |
| H | $CO_2Me$ | $CF_3$ | $CO_2CH_3$ |
| H | Ph | $CF_3$ | $CO_2CH_3$ |

| $R_2$ | $R_3$ | $R_4$ | $R_5$ |
|---|---|---|---|
| H | 4-F-Ph | $CF_3$ | $CO_2CH_3$ |
| H | 4-Cl-Ph | $CF_3$ | $CO_2CH_3$ |
| H | Me | $OCF_3$ | $CO_2CH_3$ |
| H | Et | $OCF_3$ | $CO_2CH_3$ |
| H | nPr | $OCF_3$ | $CO_2CH_3$ |
| H | iPr | $OCF_3$ | $CO_2CH_3$ |
| H | nBu | $OCF_3$ | $CO_2CH_3$ |
| H | sBu | $OCF_3$ | $CO_2CH_3$ |
| H | iBu | $OCF_3$ | $CO_2CH_3$ |
| H | tBu | $OCF_3$ | $CO_2CH_3$ |
| H | $CO_2Me$ | $OCF_3$ | $CO_2CH_3$ |
| H | Ph | $OCF_3$ | $CO_2CH_3$ |
| H | 4-F-Ph | $OCF_3$ | $CO_2CH_3$ |
| H | 4-Cl-Ph | $OCF_3$ | $CO_2CH_3$ |
| H | Me | Cl | $CO_2CH_3$ |
| H | Et | Cl | $CO_2CH_3$ |
| H | nPr | Cl | $CO_2CH_3$ |
| H | iPr | Cl | $CO_2CH_3$ |
| H | nBu | Cl | $CO_2CH_3$ |
| H | sBu | Cl | $CO_2CH_3$ |
| H | iBu | Cl | $CO_2CH_3$ |
| H | tBu | Cl | $CO_2CH_3$ |
| H | 1-pentyl | Cl | $CO_2CH_3$ |
| H | 2-pentyl | Cl | $CO_2CH_3$ |
| H | cyclopropyl | Cl | $CO_2CH_3$ |
| H | cyclobutyl | Cl | $CO_2CH_3$ |
| H | cyclopentyl | Cl | $CO_2CH_3$ |
| H | cyclohexyl | Cl | $CO_2CH_3$ |
| H | $CO_2Me$ | Cl | $CO_2CH_3$ |
| H | Ph | Cl | $CO_2CH_3$ |
| H | 4-F-Ph | Cl | $CO_2CH_3$ |
| H | 4-Cl-Ph | Cl | $CO_2CH_3$ |

| $R_2$ | $R_3$ | $R_4$ | $R_5$ |
|---|---|---|---|
| H | Me | Br | $CO_2CH_3$ |
| H | Et | Br | $CO_2CH_3$ |
| H | nPr | Br | $CO_2CH_3$ |
| H | iPr | Br | $CO_2CH_3$ |
| H | nBu | Br | $CO_2CH_3$ |
| H | sBu | Br | $CO_2CH_3$ |
| H | iBu | Br | $CO_2CH_3$ |
| H | tBu | Br | $CO_2CH_3$ |
| H | 1-pentyl | Br | $CO_2CH_3$ |
| H | 2-pentyl | Br | $CO_2CH_3$ |
| H | cyclopropyl | Br | $CO_2CH_3$ |
| H | cyclobutyl | Br | $CO_2CH_3$ |
| H | cyclopentyl | Br | $CO_2CH_3$ |
| H | cyclohexyl | Br | $CO_2CH_3$ |
| H | $CO_2Me$ | Br | $CO_2CH_3$ |
| H | Ph | Br | $CO_2CH_3$ |
| H | 4-F-Ph | Br | $CO_2CH_3$ |
| H | 4-Cl-Ph | Br | $CO_2CH_3$ |

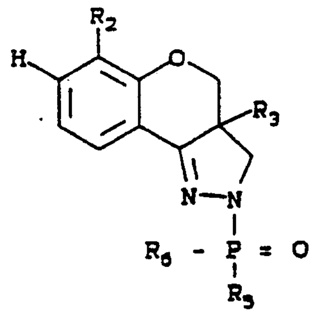

TABLE 5

| $R_2$ | $R_3$ | $R_5$ | $R_6$ |
|---|---|---|---|
| F | Me | MeO | Me |
| F | Me | MeO | Et |
| F | Me | MeO | tBu |
| F | Me | MeO | MeO |
| F | Me | EtO | Me |
| F | Me | EtO | Et |
| F | Me | EtO | tBu |
| F | Me | EtO | EtO |
| F | $CO_2Me$ | MeO | Me |
| F | $CO_2Me$ | MeO | Et |
| F | $CO_2Me$ | MeO | tBu |
| F | $CO_2Me$ | MeO | MeO |
| F | $CO_2Me$ | EtO | Me |
| F | $CO_2Me$ | EtO | Et |
| F | $CO_2Me$ | EtO | tBu |
| F | $CO_2Me$ | EtO | EtO |
| F | Ph | MeO | Me |
| F | Ph | MeO | Et |
| F | Ph | MeO | tBu |

| R2 | R3 | R5 | R6 |
|---|---|---|---|
| F | Ph | MeO | MeO |
| F | Ph | EtO | Me |
| F | Ph | EtO | Et |
| F | Ph | EtO | tBu |
| F | Ph | EtO | EtO |
| F | 4-F-Ph | MeO | Me |
| F | 4-F-Ph | MeO | Et |
| F | 4-F-Ph | MeO | tBu |
| F | 4-F-Ph | MeO | MeO |
| F | 4-F-Ph | EtO | Me |
| F | 4-F-Ph | EtO | Et |
| F | 4-F-Ph | EtO | tBu |
| F | 4-F-Ph | EtO | EtO |
| F | 4-Cl-Ph | MeO | Me |
| F | 4-Cl-Ph | MeO | Et |
| F | 4-Cl-Ph | MeO | tBu |
| F | 4-Cl-Ph | MeO | MeO |
| F | 4-Cl-Ph | EtO | Me |
| F | 4-Cl-Ph | EtO | Et |
| F | 4-Cl-Ph | EtO | tBu |
| F | 4-Cl-Ph | EtO | EtO |

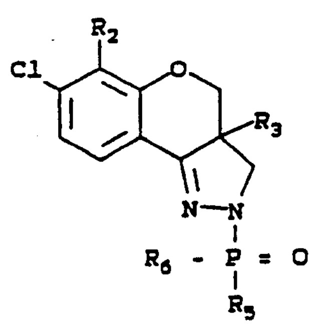

TABLE 6

| $R_2$ | $R_3$ | $R_5$ | $R_6$ |
|---|---|---|---|
| H | Me | MeO | Me |
| H | Me | MeO | Et |
| H | Me | MeO | tBu |
| H | Me | MeO | MeO |
| H | Me | EtO | Me |
| H | Me | EtO | Et |
| H | Me | EtO | tBu |
| H | Me | EtO | EtO |
| H | $CO_2Me$ | MeO | Me |
| H | $CO_2Me$ | MeO | Et |
| H | $CO_2Me$ | MeO | tBu |
| H | $CO_2Me$ | MeO | MeO |
| H | $CO_2Me$ | EtO | Me |
| H | $CO_2Me$ | EtO | Et |
| H | $CO_2Me$ | EtO | tBu |
| H | $CO_2Me$ | EtO | EtO |
| H | Ph | MeO | Me |
| H | Ph | MeO | Et |

| R2 | R3 | R5 | R6 |
|---|---|---|---|
| H | Ph | MeO | tBu |
| H | Ph | MeO | MeO |
| H | Ph | EtO | Me |
| H | Ph | EtO | Et |
| H | Ph | EtO | tBu |
| H | Ph | EtO | EtO |
| H | 4-F-Ph | MeO | Me |
| H | 4-F-Ph | MeO | Et |
| H | 4-F-Ph | MeO | tBu |
| H | 4-F-Ph | MeO | MeO |
| H | 4-F-Ph | EtO | Me |
| H | 4-F-Ph | EtO | Et |
| H | 4-F-Ph | EtO | tBu |
| H | 4-F-Ph | EtO | EtO |
| H | 4-Cl-Ph | MeO | Me |
| H | 4-Cl-Ph | MeO | Et |
| H | 4-Cl-Ph | MeO | tBu |
| H | 4-Cl-Ph | MeO | MeO |
| H | 4-Cl-Ph | EtO | Me |
| H | 4-Cl-Ph | EtO | Et |
| H | 4-Cl-Ph | EtO | tBu |
| H | 4-Cl-Ph | EtO | EtO |

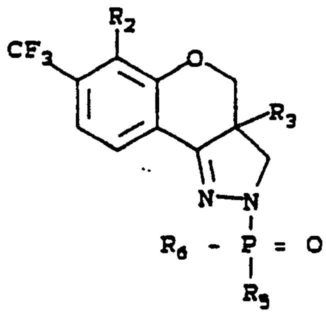

TABLE 7

| $R_2$ | $R_3$ | $R_5$ | $R_6$ |
|---|---|---|---|
| H | Me | MeO | Me |
| H | Me | MeO | Et |
| H | Me | MeO | tBu |
| H | Me | MeO | MeO |
| H | Me | EtO | Me |
| H | Me | EtO | Et |
| H | Me | EtO | tBu |
| H | Me | EtO | EtO |
| H | $CO_2Me$ | MeO | Me |
| H | $CO_2Me$ | MeO | Et |
| H | $CO_2Me$ | MeO | tBu |
| H | $CO_2Me$ | MeO | MeO |
| H | $CO_2Me$ | EtO | Me |
| H | $CO_2Me$ | EtO | Et |
| H | $CO_2Me$ | EtO | tBu |
| H | $CO_2Me$ | EtO | EtO |
| H | Ph | MeO | Me |
| H | Ph | MeO | Et |

| R2 | R3 | R5 | R6 |
|---|---|---|---|
| H | Ph | MeO | tBu |
| H | Ph | MeO | MeO |
| H | Ph | EtO | Me |
| H | Ph | EtO | Et |
| H | Ph | EtO | tBu |
| H | Ph | EtO | EtO |
| H | 4-F-Ph | MeO | Me |
| H | 4-F-Ph | MeO | Et |
| H | 4-F-Ph | MeO | tBu |
| H | 4-F-Ph | MeO | MeO |
| H | 4-F-Ph | EtO | Me |
| H | 4-F-Ph | EtO | Et |
| H | 4-F-Ph | EtO | tBu |
| H | 4-F-Ph | EtO | EtO |
| H | 4-Cl-Ph | MeO | Me |
| H | 4-Cl-Ph | MeO | Et |
| H | 4-Cl-Ph | MeO | tBu |
| H | 4-Cl-Ph | MeO | MeO |
| H | 4-Cl-Ph | EtO | Me |
| H | 4-Cl-Ph | EtO | Et |
| H | 4-Cl-Ph | EtO | tBu |
| H | 4-Cl-Ph | EtO | EtO |

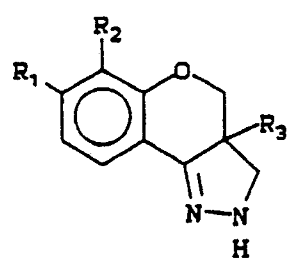

TABLE 8

| $R_1$ | $R_2$ | $R_3$ |
|---|---|---|
| Cl | H | $CO_2CH_3$ |
| Cl | H | Me* |
| Cl | H | Et |
| Cl | H | i-Pr |
| Cl | H | s-Bu |
| Cl | H | Ph |
| Cl | H | 4-F-Ph |
| Cl | H | 4-Cl-Ph |
| F | H | $CO_2CH_3$ |
| F | H | Me |
| F | H | Et |
| F | H | i-Pr |
| F | H | s-Bu |
| F | H | Ph |
| F | H | 4-F-Ph |
| F | H | 4-Cl-Ph |
| $CF_3$ | H | $CO_2CH_3$ |
| $CF_3$ | H | Me |

*The melting point of this compound is 173 to 175° (HCl Salt).

| $R_1$ | $R_2$ | $R_3$ |
|---|---|---|
| $CF_3$ | H | Et |
| $CF_3$ | H | i-Pr |
| $CF_3$ | H | s-Bu |
| $CF_3$ | H | Ph |
| $CF_3$ | H | 4-F-Ph |
| $CF_3$ | H | 4-Cl-Ph |
| H | F | $CO_2CH_3$ |
| H | F | Me |
| H | F | Et |
| H | F | i-Pr |
| H | F | s-Bu |
| H | F | Ph |
| H | F | 4-F-Ph |
| H | F | 4-Cl-Ph |
| Cl | H | $CO_2CH_3$ |
| Cl | H | Me |
| Cl | H | Et |
| Cl | H | i-Pr |
| Cl | H | s-Bu |
| Cl | H | Ph |
| Cl | H | 4-F-Ph |
| Cl | H | 4-Cl-Ph |
| H | $CF_3$ | $CO_2CH_3$ |
| H | $CF_3$ | Me |
| H | $CF_3$ | Et |
| H | $CF_3$ | i-Pr |
| H | $CF_3$ | s-Bu |
| H | $CF_3$ | Ph |
| H | $CF_3$ | 4-F-Ph |
| H | $CF_3$ | 4-Cl-Ph |

## Formulation and Use

The compounds of this invention will generally be used in formulation with an agriculturally suitable carrier comprising a liquid or solid diluent or an organic solvent. Useful formulations of the compounds of

Formula I can be prepared in conventional ways. They include dusts, granules, baits, pellets, solutions, suspensions, emulsions, wettable powders, emulsifiable concentrates, dry flowables and the like. Many of these can be applied directly. Sprayable formulations can be extended in suitable media and used at spray volumes of from about one to several hundred liters per hectare. High strength compositions are primarily used as intermediates for further formulation. The formulations, broadly, contain from less than about 1% to 99% by weight of active ingredient(s) and at least one of a) about 0.1% to 20% surfactant(s) and b) about 5% to 99% solid or liquid diluent(s). More specifically, they will contain effective amounts of these ingredients in the following approximate proportions:

| | Percent by Weight | | |
|---|---|---|---|
| | Active Ingredient | Diluent(s) | Surfactant(s) |
| Wettable Powders | 25-90 | 0-74 | 1-10 |
| Oil Suspensions, Emulsions, Solutions, (including Emulsifiable Concentrates) | 5-50 | 40-95 | 0-15 |
| Dusts | 1-25 | 70-99 | 0-5 |
| Granules, Baits and Pellets | 0.01-95 | 5-99 | 0-15 |
| High Strength Compositions | 90-99 | 0-10 | 0-2 |

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates; solution concentrates are preferably stable against phase separation at 0 °C. "McCutcheon's Detergents and Emulsifiers Annual", Allured Publ. Corp., Ridgewood, New Jersey, as well as Sisely and Wood, "Encyclopedia of Surface Active Agents", Chemical Publ. Co., Inc., New York, 1964, list surfactants and recommended uses. All formulations can contain minor amounts of additives to reduce foam, caking, corrosion, microbiological growth, etc. Preferably, ingredients should be approved by the U.S. Environmental Protection Agency for the use intended.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, U.S. 3,060,084). Granules and pellets can be made by spraying the active material upon preformed granular carriers or by agglomeration techniques. See J. E. Browning, "Agglomeration", Chemical Engineering, December 4, 1967, pages 147 and following, and "Perry's Chemical Engineer's Handbook", 4th Ed., McGraw-Hill, New York, 1963, pages 8 to 59 and following.

## Example A

| Emulsifiable Concentrate | |
|---|---|
| 6-fluoro-3a-(4-fluorophenyl)-2,3,3a,4-tetrahydro-N-[4-(trifluoromethyl)phenyl][1]benzopyrano[4,3-c]-pyrazole-2-carboxamide | 20% |
| blend of oil soluble sulfonates and polyoxyethylene ethers | 10% |
| isophorone | 70% |

The ingredients are combined and stirred with gentle warming to speed solution. A fine screen filter is included in packaging operation to insure the absence of any extraneous undissolved material in the product.

Example B

| Wettable Powder | |
|---|---|
| 6-fluoro-3a-(4-fluorophenyl)-2,3,3a,4-tetrahydro-N[4-(trifluoromethyl)phenyl][1]benzopyrano[4,3-c]-pyrazole-2-carboxamide | 30% |
| sodium alkylnaphthalenesulfonate | 2% |
| sodium ligninsulfonate | 2% |
| synthetic amorphous silica | 3% |
| kaolinite | 63% |

The active ingredient is mixed with the inert materials in a blender. After grinding in a hammer-mill, the material is re-blended and sifted through a 50 mesh screen.

Example C

| Dust | |
|---|---|
| Wettable powder of Example B | 10% |
| pyrophyllite (powder) | 90% |

The wettable powder and the pyrophyllite diluent are thoroughly blended and then packaged. The product is suitable for use as a dust.

Example D

| Granule | |
|---|---|
| 6-fluoro-3a-(4-fluorophenyl)-2,3,3a,4-tetrahydro-N-[4-(trifluoromethyl)phenyl][1]benzopyrano[4,3-c]-pyrazole-2-carboxamide | 10% |
| attapulgite granules (low volative matter, 0.71/0.30 mm; U.S.S. No. 25-50 sieves) | 90% |

The active ingredient is dissolved in a volatile solvent such as acetone and sprayed upon dedusted and pre-warmed attapulgite granules in a double cone blender. The acetone is then driven off by heating. The granules are then allowed to cool and are packaged.

Example E

| Granule | |
|---|---|
| Wettable powder of Example B | 15% |
| gypsum | 69% |
| potassium sulfate | 16% |

The ingredients are blended in a rotating mixer and water sprayed on to accomplish granulation. When most of the material has reached the desired range of 0.1 to 0.42 mm (U.S.S. No. 18 to 40 sieves), the granules are removed, dried, and screened. Oversize material is crushed to produce additional material in the desired range. These granules contain 4.5% active ingredient.

Example F

| Solution | |
|---|---|
| 7-chloro-2,3,3a,4-tetrahydro-3a-methyl-N-[4-(trifluoromethyl)phenyl][1]benzopyrano-[4,3-c]pyrazole-2-carboxamide | 25% |
| N-methyl-pyrrolidone | 75% |

The ingredients are combined and stirred to produce a solution suitable for direct, low volume application.

Example G

| Aqueous Suspension | |
|---|---|
| methyl 7-chloro-2,3,3a,4-tetrahydro-2-[[[4-(trifluoromethyl)phenyl]amino]carbonyl][1]-benzopyrano-[4,3-c]pyrazole-3a-carboxylate | 40% |
| polyacrylic acid thickener | 0.3% |
| dodecyclophenol polyethylene glycol ether | 0.5% |
| disodium phosphate | 1.0% |
| monosodium phosphate | 0.5% |
| polyvinyl alcohol | 1.0% |
| water | 56.7% |

The ingredients are blended and ground together in a sand mill to produce particles substantially all under 5 microns in size.

Example H

| Oil Suspension | |
|---|---|
| 3a-(4-chlorophenyl)-2,3,3a,4-tetrahydro-N-[4(trifluoromethyl)phenyl][1]benzopyrano[4,3-c]pyrazole-2-carboxamide | 35.0% |
| blend of polyalcohol carboxylic esters and oil soluble petroleum sulfonates | 6.0% |
| xylene range solvent | 59.0% |

The ingredients are combined and ground together in a sand mill to produce particles substantially all below 5 microns. The product can be used directly, extended with oils, or emulsified in water.

Example I

| Bait Granules | |
|---|---|
| 6-fluoro-2,3,3a,4-tetrahydro-3a-phenyl-N-[4-trifluoromethyl)phenyl][1]benzopyrano[4,3-c]pyrazole-2-carboxamide | 3.0% |
| blend of polyethoxylated nonylphenols and sodium dodecylbenzene sulfonates | 9.0% |
| ground up corn cobs | 88.0% |

The active ingredient and surfactant blend are dissolved in a suitable solvent such as acetone and sprayed onto the ground, corn cobs. The granules are then dried and packaged.

Compounds of Formula I can also be mixed with one or more other insecticides, fungicides, nematocides, bactericides, acaricides, or other biologically active compounds to form a multi-component pesticide giving an even broader spectrum of effective agricultural protection. Examples of other agricultural protectants with which compounds of this invention can be formulated are:

Insecticides:

3-hydroxy-N-methylcrotonamide(dimethylphosphate)ester (monocrotophos)
methylcarbamic acid, ester with 2,3-dihydro-2,2-dimethyl-7-benzofuranol (carbofuran)
O-[2,4,5-trichloro-α-(chloromethyl)benzyl]phosphoric acid, O′,O′-dimethyl ester (tetrachlorvinphos)
2-mercaptosuccinic acid, diethyl ester, S-ester with thionophosphoric acid, dimethyl ester (malathion)
phosphorothioic acid, O,O-dimethyl, O-p-nitrophenyl ester (methyl parathion)
methylcarbamic acid, ester with α-naphthol (carbaryl)
methyl O-(methylcarbamoyl)thiolacetohydroxamate (methomyl)
N′-(4-chloro-o-tolyl)-N,N-dimethylformamidine (chlordimeform)
O,O-diethyl-O-(2-isopropyl-4-methyl-6-pyrimidylphosphorothioate (diazinon)
octachlorocamphene (toxaphene)
O-ethyl O-p-nitrophenyl phenylphosphonothioate (EPN)
(S)-a-cyano-m-phenoxybenzyl(1R,3R)-3-(2,2-dibromovinyl)-2,2-dimethylcyclopropanecarboxylate (deltamethrin)
Methyl-N′,N′-dimethyl-N-[(methylcarbamoyl)oxy]-1-thioox amimidate (oxamyl)
cyano(3-phenoxyphenyl)-methyl-4-chloro-a-(1-methylethyl)benzeneacetate (fenvalerate)
(3-phenoxyphenyl)methyl(±)-cis,trans-3-(2,2-dichloro ethenyl)-2,2-dimethylcyclopropanecarboxylate (permethrin)
a-cyano-3-phenoxybenzyl 3-(2,2-dichlorovinyl)-2,2-dimethylcyclopropane carboxylate (cypermethrin)
O-ethyl-S-(p-chlorophenyl)ethylphosphonodithioate (profenofos)
phosphorothiolothionic acid,
O-ethyl-O-[4-(methylthio)-phenyl]-S-n-propyl ester (sulprofos).

Additional insecticides are listed hereafter by their common names: triflumuron, diflubenzuron, methoprene, buprofezin, thiodicarb, acephate, azinphosmethyl, chlorpyrifos, dimethoate, fonophos, isofenphos, methidathion, methamidiphos, monocrotphos, phosmet, phosphamidon, phosalone, pirimicarb, phorate, profenofos, terbufos, trichlorfon, methoxychlor, bifenthrin, biphenate, cyfluthrin, fenpropathrin, fluvalinate, flucythrinate, tralomethrin, metaldehyde and rotenone.

Fungicides:

methyl 2-benzimidazolecarbamate (carbendazim)
tetramethylthiuram disulfide (thiuram)
n-dodecylguanidine acetate (dodine)
manganese ethylenebisdithiocarbamate (maneb)
1,4-dichloro-2,5-dimethoxybenzene (chloroneb)
methyl 1-(butylcarbamoly)-2-benzimidazolecarbamate (benomyl)
1-[2-(2,4-dichlorophenyl)-4-propyl-1,3-dioxolan-2-ylmethyl]-1H-1,2,4-triazole (propiconazole)
2-cyano-N-ethylcarbamoy-2-methoxyiminoacetamide (cymoxanil)
1-(4-chlorophenoxy)-3,3-dimethyl-1-(1H-1,2,4-triazol-1-yl)-2-butanone (triadimefon)
N-(trichloromethylthio)tetrahydrophthalimide (captan)
N-(trichloromethylthio)phthalimide (folpet)
1-[[[bis(4-fluorophenyl)][methyl]silyl]methyl]-1H-1,2,4-triazole.

Nematocides:

S-methyl 1-(dimethylcarbamoyl)-N-(methylcarbamoyloxy)thioformimidate

S-methyl
1-carbamoyl-N-(methylcarbamoyloxy)thioformimidate
N-isopropylphosphoramidic acid, O-ethyl
O′-[4-(methylthio)-m-tolyl]diester (fenamiphos)

Bactericides:

tribasic copper sulfate
streptomycin sulfate

Acaricides:

senecioic acid, ester with 2-sec-butyl-4,6-dinitrophenol (binapacryl)
6-methyl-1,3-cithiolo[4,5-$\beta$]quinoxalin-2-one (oxythioquinox)
ethyl 4,4′-dichlorobenzilate (chlorobenzilate)
1,1-bis(p-chlorophenyl)-2,2,2-trichloroethanol (dicofol)
bis(pentachloro-2,4-cyclopentadien-1-yl) (dienochlor)
tricyclohexyltin hydroxide (cyhexatin)
trans-5-(4-chlorophenyl)-N-cyclohexyl-4-methyl-2-oxothiazolidine-3-carboxamide (hexythiazox)
amitraz
propargite
fenbutatin-oxide

Biological

Bacillus thuringiensis
Avermectin B.

Utility

The compounds of this invention exhibit activity against a wide spectrum of foliar and soil inhabiting arthropods which are pests of growing and stored agronomic crops, forestry, greenhouse crops, ornamentals, nursery crops, stored food and fiber products, livestock, household, and public and animal health. Those skilled in the art will recognize that not all compounds are equally effective against all pests but the compounds of this invention display activity against economically important agronomic, forestry, greenhouse, ornamental food and fiber product, stored product, domestic structure, and nursery pests, such as:
larvae of the order Lepidoptera including fall and beet armyworm and other Spodoptera spp., tobacco budworm, corn earworm and other Heliothis spp., European corn borer, navel orangeworm, stalk/stem borers and other pyralids, cabbage and soybean loopers and other loopers, codling moth, grape berry moth and other tortricids, black cutworm, spotted cutworm, other cutworms and other noctuids, diamondback moth, green cloverworm, velvetbean caterpillar, green cloverworm, pink bollworm, gypsy moth, and spruce budworm;
foliar feeding larvae and adults of the order Coleoptera including Colorado potato beetle, Mexican bean beetle, flea beetle, Japanese beetles, and other leaf beetles, boll weevil, rice water weevil, granary weevil, rice weevil and other weevil pests, and soil inhabiting insects such as Western corn rootworm and other Diabrotica spp., Japanese beetle, European chafer and other coleopteran grubs, and wireworms;
adults and larvae of the orders Hemiptera and Homoptera including tarnished plant bug and other plant bugs (miridae), aster leafhopper and other leafhoppers (cicadellidae), rice planthopper, brown planthopper, and other planthoppers (fulgoroidea), psylids, whiteflies (aleurodidae), aphids (aphidae), scales (coccidae and diaspididae), lace bugs (tingidae), stink bugs (pentatomidae), cinch bugs and other seed bugs (lygaeidae), cicadas (cicadidae), spittlebugs (cercopids), squash bugs (coreidae), red bugs and cotton stainers (pyrrhocoridae);
adults and larvae of the order acari (mites) including European red mite, two spotted spider mite, rust mites, McDaniel mite, and foliar feeding mites;

adults and immatures of the order Orthoptera including grasshoppers;
adults and immatures of the order Diptera including leafminers, midges, fruit flies (tephritidae), and soil maggots;
adults and immatures of the order Thysanoptera including onion thrips and other foliar feeding thrips.

The compounds are also active against economically important livestock, household, public and animal health pests such as:
insect pests of the order Hymenoptera including carpenter ants, bees, hornets, and wasps;
insect pests of the order Diptera including house flies, stable flies, face flies, horn flies, blow flies, and other muscoid fly pests, horse flies, deer flies and other Brachycera, mosquitoes, black flies, biting midges, sand flies, sciarids, and other Nematocera;
insect pests of the order Orthoptera including cockroaches and crickets;
insect pests of the order Isoptera including the Eastern subterranean termite and other termites;
insect pests of the order Mallophaga and Anoplura including the head louse, body louse, chicken head louse and other sucking and chewing parasitic lice that attack man and animals;
insect pests of the order Siphonoptera including the cat flea, dog flea and other fleas.

The specific species for which control is exemplified are: fall armyworm, Spodoptera fruigiperda; tobacco budworm, Heliothis virescens; boll weevil, Anthonomus grandis; aster leafhopper, Macrosteles fascifrons; black bean aphid, (Aphis Fabae); southern corn rootworm, Diabrotica undecimpunctata. The pest control protection afforded by the compounds of the present invention is not limited, however, to these species. The compounds of this invention may also be utilized as rodenticides.

## Application

Arthropod pests are controlled and protection of agronomic crops, animal and human health is achieved by applying one or more of the Formula I compounds of this invention, in an effective amount, to the environment of the pests including the agronomic and/or nonagronomic locus of infestation, to the area to be protected, or directly on the pests to be controlled. Because of the diversity of habitat and behavior of these arthropod pest species, many different methods of application are employed. A preferred method of application is by spraying with equipment that distributes the compound in the environment of the pests, on the foliage, animal, person, or premise, in the soil or animal, to the plant part that is infested or needs to be protected. Alternatively, granular formulations of these toxicant compounds can be applied to or incorporated into the soil. Other methods of application can also be employed including direct and residual sprays, aerial sprays, baits, eartags, boluses, foggers, aerosols, and many others. The compounds can be incorporated into baits that are consumed by the arthropods or in devices such as traps and the like which entice them to ingest or otherwise contact the compounds.

The compounds of this invention can be applied in their pure state, but most often application will be of a formulation comprising one or more compounds with suitable carriers, diluents, and surfactants and possibly in combination with a food depending on the contemplated end use. A preferred method of application involves spraying a water dispersion or refined oil solution of the compounds. Combinations with spray oils, spray oil concentrations, and synergists such as piperonyl butoxide often enhance the efficacy of the compounds of Formula I.

The rate of application of the Formula I compounds required for effective control will depend on such factors as the species of arthropod to be controlled, the pest's life cycle, life stage, its size, location, time of year, host crop or animal, feeding behavior, mating behavior, ambient moisture, temperature, etc. In general, application rates of 0.01 to 2 kg of active ingredient per hectare are sufficient to provide large-scale effective control of pests in agronomic ecosystems under normal circumstances, but as little as 0.001 kg/hectare or as much as 8 kg hectare may be employed. Preferred rates are from about 0.01 to 0.1 kg per hectare. For nonagronomic applications, effective use rates will range from about 0.1 to 5 mg/square foot but as little as about 0.01 mg/square foot or as much as 15 mg/square foot may be required.

The following Examples demonstrate the control efficacy of compounds of Formula I on specific pests; see Table A for compound descriptions.

TABLE A

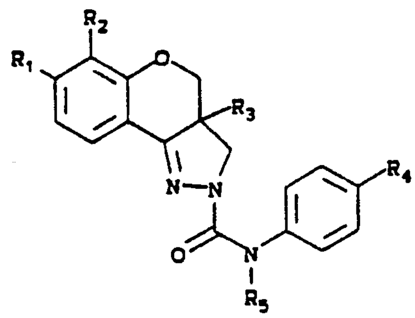

| Cmpd. | $R_1$ | $R_2$ | $R_3$ | $R_4$ | $R_5$ | m.p.(°C) |
|---|---|---|---|---|---|---|
| 1 | H | F | $CO_2CH_3$ | $CF_3$ | H | 217-220 |
| 2 | H | F | Ph | $CF_3$ | H | 227-228 |
| 3 | H | F | 4-F-Ph | $CF_3$ | H | 185-187 |
| 4 | H | F | 4-Cl-Ph | $CF_3$ | H | 189-190 |
| 5 | Cl | H | $CH_3$ | $CF_3$ | H | 179-180 |
| 6 | Cl | H | $CO_2CH_3$ | $CF_3$ | H | 214-215 |
| 7 | Cl | H | Ph | $CF_3$ | H | 192-193 |
| 8 | Cl | H | 4-F-Ph | $CF_3$ | H | 231-232 |
| 9 | Cl | H | 4-Cl-Ph | $CF_3$ | H | 222-224 |
| 10 | Cl | H | 4-Cl-Ph | $CF_3$ | $COCH_3$ | 103-106 |
| 11 | H | F | 4-Cl-Ph | Cl | H | 235-239 |
| 12 | H | F | 4-Cl-Ph | Br | H | 228-130 |
| 13 | H | F | 4-F-Ph | $CF_3$ | $COCH_3$ | 155-156 |
| 14 | F | H | $CH_3$ | $CF_3$ | H | 144-146 |
| 15 | F | H | $CH_3$ | Cl | H | >250 |
| 16 | F | H | $CH_3$ | Br | H | 145-149 |
| 17 | F | H | $CO_2CH_3$ | $CF_3$ | H | 200-202 |
| 18 | F | H | Ph | $CF_3$ | H | 206-208 |
| 19 | $CF_3$ | H | $CO_2CH_3$ | $CF_3$ | H | 189-190 |
| 20 | Cl | H | Ph | $CF_3$ | $COCH_3$ | 195-197 |
| 21 | F | H | $CO_2CH_3$ | $CF_3$ | $COCH_3$ | 158-160 |

| Cmpd. | $R_1$ | $R_2$ | $R_3$ | $R_4$ | $R_5$ | m.p.(°C) |
|---|---|---|---|---|---|---|
| 22 | Cl | H | $CH_3$ | $CF_3$ | $COCH_3$ | 63-70 |
| 23 | H | F | $CH_3$ | $CF_3$ | H | 192-194 |
| 24 | Cl | H | $CH_3$ | $OCF_3$ | H | 132-135 |
| 25 | Cl | H | $CO_2CH_3$ | $OCF_3$ | H | 195-197 |
| 26 | Cl | H | $CH_3$ | Cl | H | 165-169 |
| 27 | Cl | H | $CH_3$ | Br | H | 161-162 |
| 28 | Cl | H | $CO_2CH_3$ | Br | H | 211-215 |
| 29 | Cl | H | $CO_2CH_3$ | $CF_3$ | $COCH_3$ | 198-200 |
| 30 | Cl | H | iPr | $CF_3$ | H | 150-152 |
| 31 | Cl | H | iPr | Br | H | 154-155 |
| 32 | Cl | H | iPr | $OCF_3$ | H | 167-168 |
| 33 | Cl | H | iPr | Cl | H | 152-153 |
| 34 | Cl | H | Et | $CF_3$ | H | 155-157 |
| 35 | Cl | H | Et | $OCF_3$ | H | 120-122 |
| 36 | $CF_3$ | H | $CO_2CH_3$ | $OCF_3$ | H | 161-163 |
| 37 | $CF_3$ | H | iPr | $CF_3$ | H | 160-161 |
| 38 | $CF_3$ | H | $CH_3$ | $CF_3$ | H | 177-179 |
| 39 | $CF_3$ | H | iPr | $OCF_3$ | H | 148-149 |
| 40 | $CF_3$ | H | $CH_3$ | $OCF_3$ | H | oil |
| 41 | $CF_3$ | H | $CO_2CH_3$ | $CF_3$ | $COCH_3$ | 180-181 |
| 42 | Cl | H | iPr | CF3 | $COCH_3$ | 115-116 |
| 43 | $CF_3$ | H | $CO_2CH_3$ | $CF_3$ | $CO_2CH_3$ | 157-159 |
| 44 | Cl | H | $CO_2CH_3$ | $CF_3$ | $CO_2CH_3$ | 218-220 |
| 45 | Cl | H | $CH_3$ | $CF_3$ | $CO_2CH_3$ | 112-115 |
| 46 | H | F | 4-F-Ph | $CF_3$ | $CO_2CH_3$ | 100-104 |
| 47 | Cl | H | sec-Bu | $CF_3$ | H | 177-178 |
| 48 | Cl | H | t-Bu | $CF_3$ | H | 181-183 |
| 49 | Cl | H | i-Pr | $CF_3$ | $CO_2CH_3$ | 180-181 |
| 50 | Cl | H | sec-Bu | $CF_3$ | $CO_2CH_3$ | 158-159 |
| 51 | $CF_3$ | H | $CO_2CH_3$ | Cl | H | 208-210 |
| 52 | $CF_3$ | H | $CO_2CH_3$ | Br | H | 201-202 |
| 53 | $CF_3$ | H | 4-F-Ph | $CF_3$ | H | 216-217 |
| 54 | $CF_3$ | H | 4-F-Ph | Cl | H | 195-197 |

## EXAMPLE 7

### Fall Armyworm

Test units, each consisting of an 8-ounce plastic cup containing a layer of wheat germ diet, approximately 0.5 cm thick, were prepared. Ten third-instar larvae of fall armyworm (Spodoptera frugiperda) were placed into each cup. Solutions of each of the test compounds (acetone/distilled water 75/25 solvent) were sprayed onto the cups, a single solution per set of three cups. Spraying was accomplished by passing the cups, on a conveyer belt, directly beneath a flat fan hydraulic nozzle which discharged the spray at a rate of 0.5 pounds of active ingredient per acre (about 0.55 kg/ha) at 30 p.s.i. The cups were then covered and held at 27° C and 50% relative humidity for 72 hours, after which time readings were taken. The results are tabulated below.

| Compound | % Mortality | Compound | % Mortality |
|---|---|---|---|
| 1 | 100 | 23 | 100 |
| 2 | 100 | 26 | 100 |
| 3 | 100 | 27 | 100 |
| 4 | 100 | 28 | 100 |
| 5 | 100 | 30 | 100 |
| 6 | 100 | 31 | 100 |
| 7 | 100 | 32 | 100 |
| 8 | 100 | 33 | 100 |
| 9 | 100 | 34 | 100 |
| 10 | 100 | 35 | 100 |
| 11 | 100 | 43 | 100 |
| 12 | 100 | 44 | 100 |
| 13 | 100 | 45 | 100 |
| 14 | 100 | 46 | 100 |
| 15 | 100 | 47 | 100 |
| 17 | 100 | 48 | 100 |
| 18 | 100 | 49 | 100 |
| 19 | 100 | 50 | 100 |
| 20 | 100 | 51 | 100 |
| | | 52 | 100 |
| | | 53 | 100 |
| | | 54 | 100 |

## EXAMPLE 8

### Tobacco Budworm

The test procedure of Example 7 was repeated for efficacy against third-instar larvae of the tobacco budworm (Heliothis virescens) except that mortality was assessed at 48 hours. The results are tabulated below.

| Compound | % Mortality | Compound | % Mortality |
|---|---|---|---|
| 1 | 93 | 23 | 67 |
| 2 | 80 | 26 | 100 |
| 3 | 100 | 27 | 100 |
| 4 | 100 | 28 | 60 |
| 5 | 100 | 30 | 100 |
| 6 | 100 | 31 | 100 |
| 7 | 93 | 32 | 100 |
| 8 | 100 | 33 | 100 |
| 9 | 100 | 34 | 100 |
| 10 | 100 | 35 | 100 |
| 11 | 100 | 43 | 100 |
| 12 | 100 | 44 | 67 |
| 13 | 100 | 45 | 0 |
| 14 | 100 | 46 | 100 |
| 17 | 93 | 47 | 100 |
| 18 | 93 | 48 | 100 |
| 19 | 100 | 49 | 0 |
| 20 | 100 | 50 | 100 |
| | | 51 | 0 |
| | | 52 | 100 |
| | | 53 | 100 |
| | | 54 | 100 |

## EXAMPLE 9

Southern Corn Rootworm

Test units, each consisting of an 8-ounce plastic cup containing 1 sprouted corn seed, were prepared. Sets of three test units were sprayed as described in Example 7 with individual solutions of the test compounds. After the spray on the cups had dried, five third-instar larvae of the southern corn rootworm (Diabrotica undecimpunctata howardi) were placed into each cup. A moistened dental wick was inserted into each cup to prevent drying and the cups were then covered. The cups were then held at 27° C and 50% relative humidity for 48 hours, after which time mortality readings were taken. Of the compounds tested on Southern rootworm, the following tabulation depicts the activity:

| Compound | % Mortality | Compound | % Mortality |
|---|---|---|---|
| 1 | 100 | 27 | 100 |
| 2 | 100 | 28 | 100 |
| 3 | 100 | 30 | 100 |
| 4 | 100 | 31 | 100 |
| 5 | 100 | 32 | 100 |
| 6 | 100 | 33 | 100 |
| 7 | 100 | 34 | 100 |
| 8 | 100 | 35 | 100 |
| 9 | 100 | 43 | 100 |
| 10 | 100 | 44 | 100 |
| 11 | 100 | 45 | 100 |
| 12 | 100 | 46 | 0 |
| 13 | 100 | 47 | 100 |
| 14 | 100 | 48 | 100 |
| 17 | 100 | 47 | 100 |
| 18 | 100 | 49 | 100 |
| 19 | 100 | 50 | 0 |
| 20 | 100 | 51 | 100 |
| 21 | 100 | 52 | 100 |
| 26 | 100 | 53 | 100 |
| | | 54 | 100 |

## EXAMPLE 10

### Boll Weevil

Five adult boll weevils (Anthonomus grandis) were placed into each of a series of 9-ounce cups. The test procedure employed was then otherwise the same as in Example 7. Mortality readings were taken 48 hours after treatment. Of the compounds tested on boll weevil, the following tabulation depicts the activity:

| Compound | % Mortality | Compound | % Mortality |
|---|---|---|---|
| 1 | 100 | 23 | 100 |
| 2 | 100 | 26 | 100 |
| 3 | 100 | 27 | 100 |
| 4 | 100 | 28 | 100 |
| 5 | 0 | 30 | 100 |
| 6 | 100 | 31 | 100 |
| 7 | 100 | 32 | 100 |
| 8 | 73 | 33 | 100 |
| 9 | 87 | 34 | 100 |
| 10 | 100 | 35 | 100 |
| 11 | 100 | 43 | 100 |
| 12 | 100 | 44 | 100 |
| 13 | 100 | 45 | 100 |
| 14 | 100 | 46 | 100 |
| 17 | 100 | 47 | 100 |
| 18 | 100 | 48 | 87 |
| 19 | 100 | 49 | 73 |
| 20 | 100 | 50 | 100 |
| | | 51 | 100 |
| | | 52 | 100 |
| | | 53 | 100 |
| | | 54 | 100 |

## EXAMPLE 11

### Aster Leafhopper

Test units were prepared from a series of 12-ounce cups, each containing oat (Avena sativa) seedlings in a 1-inch layer of sterilized soil. The test units were sprayed as described in Example 7 with individual solutions of the below-listed compounds. After the oats had dried from the spraying, between 10 and 15 adult aster leafhoppers (Mascrosteles fascifrons) were aspirated into each of the covered cups. The cups were held at 27° C and 50% relative humidity for 48 hours, after which time mortality readings were taken. Of the compounds tested on aster leafhopper, the following tabulation depicts the activity:

| Compound | % Mortality | Compound | % Mortality |
|---|---|---|---|
| 1 | 97 | 23 | 78 |
| 2 | 100 | 26 | 100 |
| 3 | 100 | 27 | 100 |
| 4 | 98 | 28 | 100 |
| 5 | 100 | 30 | 100 |
| 6 | 95 | 31 | 100 |
| 7 | 72 | 32 | 100 |
| 8 | 74 | 33 | 100 |
| 9 | 48 | 34 | 100 |
| 10 | 76 | 35 | 100 |
| 11 | 100 | 43 | 100 |
| 12 | 100 | 44 | 100 |
| 13 | 100 | 45 | 100 |
| 14 | 100 | 46 | 100 |
| 17 | 100 | 47 | 100 |
| 18 | 100 | 48 | 100 |
| 19 | 100 | 49 | 100 |
| 20 | 100 | 50 | 100 |
| | | 51 | 100 |
| | | 52 | 100 |
| | | 53 | 88 |
| | | 54 | 100 |

## EXAMPLE 12

### Black Bean Aphid

Test units were prepared, each consisting of a single nasturtium (Tropaeolum sp.) leaf infested with 5 to 10 black bean aphids (Aphis fabae). Each leaf was cut and individually held in a 1-dram glass vial filled with a 10% sugar solution prior to and after spraying. During spraying, the infested leaves were inverted to expose the aphids, and each leaf was held in place by a clip. Three test units with leaves were sprayed with individual solutions of the below-listed compounds following the spray procedure of Example 7. Sprayed leaves were returned to the individual glass vials, covered with a clear plastic 1-ounce cup and were held at 27°C and 50% relative humidity for 48 hours, after which time mortality readings were taken. The results are tabulated below.

| Compound | % Mortality | Compound | % Mortality |
|---|---|---|---|
| 1 | 52 | 23 | 24 |
| 2 | 30 | 30 | 99 |
| 4 | 54 | 31 | 100 |
| 5 | 71 | 34 | 71 |
| 6 | 24 | 35 | 73 |
| 8 | 62 | 43 | 79 |
| 9 | 30 | 47 | 86 |
| 12 | 35 | 53 | 45 |
| 13 | 73 | 54 | 44 |
| 14 | 86 | | |
| 15 | 36 | | |
| 16 | 23 | | |
| 19 | 64 | | |
| 20 | 79 | | |

## EXAMPLE 13

Two-Spotted Spider Mite

Test units, each consisting of one-inch square sections of kidney bean leaves, were infested with 20 to 30 adult two-spotted spider mites (Tetranychus urticae) and sprayed as described in Example 7 with individual solutions of the below-listed compounds. Three test units were sprayed per solution. Sprayed leaf sections were then placed on a layer of moistened cotton in a Petri dish and held at 27° C and 50% relative humidity for 48 hours, after which time mortality readings were taken. The results are tabulated below.

| Compound | % Mortality | Compound | % Mortality |
|---|---|---|---|
| 1 | 12 | 20 | 6 |
| 2 | 71 | 23 | 89 |
| 3 | 14 | 43 | 26 |
| 4 | 13 | 44 | 7 |
| 8 | 12 | 45 | 24 |
| 9 | 7 | 46 | 3 |
| 12 | 16 | 47 | 9 |
| 13 | 18 | 48 | 3 |
| 14 | 60 | 49 | 0 |
| 19 | 37 | 50 | 0 |

In Examples 7 to 13, Compounds 7, 8, 9, 12, 20, 53 and 54 were tested at 0.14 kg per hectare and Compounds 15 and 16 were tested at 0.028 kg per hectare. Only one test unit was used for compounds 12, 15, 16, 20, 53 and 54.

**Claims**

1. A compound of the formula

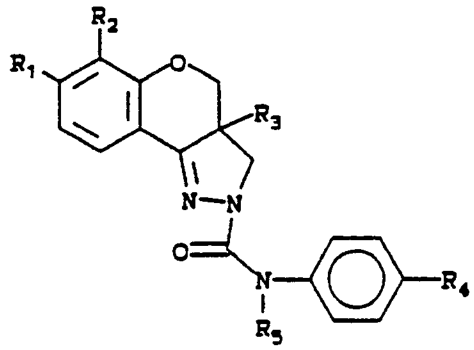

(I)

wherein

$R_1$ is selected from H, F, Cl and $CF_3$;

$R_2$ is selected from H and F, one of $R_1$ or $R_2$ being H but not both;

$R_3$ is selected from $C_1$ to $C_6$ alkyl, $C_3$ to $C_6$ cycloalkyl, $C_4$ to $C_7$ cycloalkylalkyl, $CO_2CH_3$, phenyl optionally substituted with F or Cl in the para-position and benzyl optionally substituted with F or Cl in the para-position;

$R_4$ is selected from Cl, Br, $CF_3$ and $OCF_3$; and

$R_5$ is selected from H, $C(O)CH_3$ and $C(O)OCH_3$;

provided that:

(i) when $R_1$ is F, $R_4$ is Br and $R_5$ is H, then $R_3$ is other than $CO_2CH_3$; and

(ii) when $R_1$ is Cl, $R_4$ is Cl and $R_5$ is H, then $R_3$ is other than n-propyl.

2. A compound according to Claim 1 wherein $R_3$ is selected from $C_1$-$C_6$ alkyl, $CO_2CH_3$, phenyl optionally substituted with F or Cl in the para-position, and benzyl optionally substituted with F or Cl in the para-position.

3. A compound according to Claim 2 wherein $R_3$ is selected from $C_1$-$C_6$ alkyl, $CO_2CH_3$, and phenyl optionally substituted with F or Cl in the para-position.

4. A compound according to Claim 1 wherein $R_3$ is selected from $C_3$-$C_6$ cycloalkyl and $C_4$-$C_7$ cycloalkylalkyl.

5. A compound according to Claim 2 wherein $R_3$ is selected from $CH_3$, isopropyl, and $CO_2CH_3$; and $R_4$ is selected from $CF_3$, $OCF_3$, and $R_5$ = H.

6. A compound according to Claim 5:
methyl 7-chloro-2,3,3a,4-tetrahydro-2-[[[4-(trifluoromethyl)phenyl]amino]carbonyl][1]benzopyrano-[4,3-c]-pyrazole-3a-carboxylate.

7. A compound according to Claim 5:
7-chloro-2,3,3a,4-tetrahydro-3a-methyl-N-[4-(trifluoromethyl)phenyl][1]benzopyrano-[4,3-c]pyrazole-2-carboxamide.

8. A compound according to Claim 5:
7-chloro-2,3,3a,4-tetrahydro-3a-(1-methylethyl)-N-[4-(trifluoromethyl)phenyl][1]benzopyrano[4,3-c]pyrazole-2-carboxamide.

9. A compound according to Claim 5:
6-fluoro-3a-(4-fluorophenyl)-2,3,3a,4-tetrahydro-N-[4-(trifluoromethyl)phenyl][1]benzopyrano[4,3-c]pyrazole-2-carboxamide.

10. A compound according to Claim 5:
methyl 2,3,3a,4-tetrahydro-7-(trlfluoromethyl)-2-[[[4-(trifluoromethyl)phenyl]amino]carbonyl][1]benzopyrano-[4,3-c]pyrazole-3a-carboxylate.

11. A compound according to Claim 5:
methyl 2,3,3a,4-tetrahydro-7-(trifluoromethyl)-2-[[[4-(trifluoromethoxy)phenyl]amino]carbonyl][1]benzopyrano-[4,3-c]pyrazole-3a-carboxylate.

12. A compound of the formula

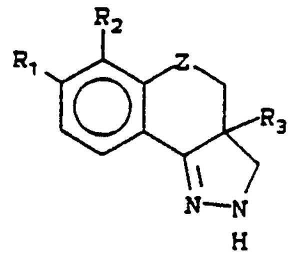

wherein:
$R_1$ is selected from H, F, Cl and $CF_3$;
$R_2$ is selected from H and F, one of $R_1$ or $R_2$ being H but not both;
$R_3$ is selected from $C_1$ to $C_6$ alkyl, $C_3$ to $C_6$ cycloalkyl, $C_4$ to $C_7$ cycloalkylalkyl, $CO_2CH_3$, phenyl optionally substituted with F or Cl in the para-position and benzyl optionally substituted with F or Cl in the para-position; and
Z is $CH_2$ or O.

13. An arthropodicidal composition comprising an arthropodicidally effective amount of a compound according to any one of Claims 1 to 11 and a carrier therefor.

14. A method for controlling arthropods comprising applying to them or to their environment an arthropodicidally effective amount of a compound according to any one of Claims 1 to 11.

15. A method for making a compound according to Claim 1 comprising the steps:
(i) reacting a compound of the formula

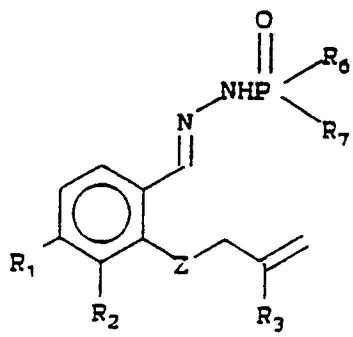

with base and a halogenating agent to form a compound of the formula

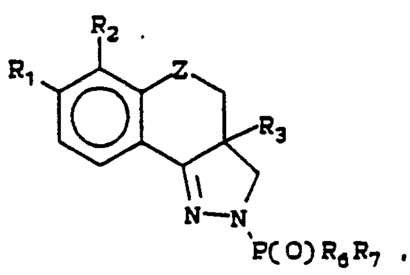

(ii) reacting the compound formed in step (i) with a strong acid to form a compound of the formula

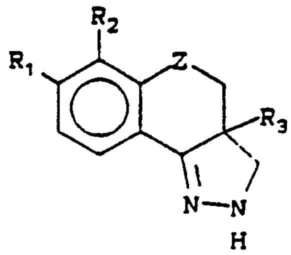

and

(iii) reacting the compound formed in step (ii) with a compound of the formula

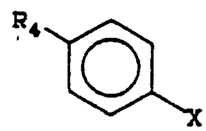

wherein:

$R_6$ and $R_7$ are independently selected from $C_1$ to $C_4$ alkyl, $C_1$ to $C_4$ alkoxy, and phenyl optionally substituted with $C_1$ to $C_3$ alkyl or halogen, or, $R_6$ and $R_7$ can be taken together to form a 5- or 6-membered ring optionally substituted with $C_1$ to $C_2$ alkyl;

Z is $CH_2$ or O; and

X is NCO or NHCOF.